# EUROPEAN PATENT APPLICATION

(11) **EP 1 291 023 A1**
(43) Date of publication of application: **12.03.2003**
(21) Application number: 01938630.9
(22) Date of filing: 13.06.2001
(51) Int. Cl.: A61K 47/34, A61K 9/50, A61K 9/52, A61K 31/4436, A61K 31/519, C07D 495/04, A61K 38/09, A61K 45/00, A61P 5/24

(54) **SUSTAINED RELEASE COMPOSITIONS**

(30) Priority: 14.06.2000 JP 2000178534
(71) Applicant: Takeda Chemical Industries, Ltd., Osaka-shi, Osaka 541-0045 (JP)
(72) Inventor: HATA, Yoshio, Ibaraki-shi, Osaka 567-0047 (JP); YAMAGATA, Yutaka, Kobe-shi, Hyogo 654-0122 (JP); IGARI, Yasutaka, Kobe-shi, Hyogo 658-0015 (JP)
(74) Representative: von Kreisler, Alek, Dipl.-Chem.
(86) International application number: JP0105009
(87) International publication number: WO01095940

(57) **Abstract**

A composition prepared by containing or blending a physiologically active non-peptide substance and a biodegradable polymer having two or more carboxylic groups at its end or a salt thereof features: (1) larger content of the physiologically active non-peptide substance can be contained, as well as release of the same can be controlled or accelerated, whereby secure pharmaceutical effect is achieved; (2) when the physiologically active non-peptide substance causes subcutaneous stimulation, an activity of canceling the stimulation by strongly acidic group at its end is expected; and (3) high glass transition point and high stability.

## Description

### TECHNICAL FIELD

The present invention relates to a composition comprising a physiologically active non-peptide substance and a biodegradable polymer having two or more carboxylic groups at its end or a salt thereof.

### BACKGROUND ART

As formulations which continuously release drugs with the aid of biodegradable polymers, the following have been reported, for example.
1) Pharmaceutical composition comprising a non-peptide bone formation promoting active agent and a biodegradable high polymer (JP 9-263545).
2) Sustained release formulation comprising a compound having angiotensin II antagonistic effect or the like and a biodegradable polymer (JP 11-315034).
3) Composition comprising a polyester including one or more free COOH group(s) ionic-bonded to a biologically active polypeptide having at least one effective ionogen amine, wherein at least 50% by weight of polypeptide existing in the composition is ionic-bonded to the polyester [WO 94/15587 (JP 8-505395)].

Although systems for controlling release of physiologically active non-peptide substances have been studied, it is still impossible to control release speed, and in particular, effective means for accelerating release have not been found yet.

### DISCLOSURE OF THE INVENTION

In making intense researches for solving the aforementioned problems, inventors of the present invention produced, for the first time, a composition in which a physiologically active non-peptide substance and a biodegradable polymer having two or more carboxylic groups at its end or a salt thereof are blended. Surprisingly, it was found that the release of the physiologically active non-peptide substance can be adjusted, and in particular, when the physiologically active non-peptide substance is water-insoluble or slightly water-soluble, the release of the physiologically active non-peptide substance is accelerated. On the basis of these findings, the present invention was accomplished.

That is, the present invention provides:
(1) a composition comprising a physiologically active non-peptide substance and a biodegradable polymer having two or more carboxylic groups at its end or a salt thereof;
(2) a composition in which a physiologically active non-peptide substance and a biodegradable polymer having two or more carboxylic groups at its end or a salt thereof are blended;
(3) the composition described in the aforementioned (2), wherein the glass transition point (Tg) of the composition is about 10°C or more higher than that of the biodegradable polymer having two or more carboxylic groups at its end;
(4) the composition described in the aforementioned (1) or (2), wherein the biodegradable polymer having two or more carboxylic groups at its end is a polymer having an α,α-dicarboxylic group or an α,β,β'-tricarboxylic group at its end;
(5) the composition described in the aforementioned (1) or (2), wherein the biodegradable polymer having two or more carboxylic groups at its end is a poly α-hydroxycarboxylic acid having an α,α-dicarboxylic group or an α,β,β'-tricarboxylic group at its end;
(6) the composition described in the aforementioned (5), wherein the poly α-hydroxycarboxylic acid is linear poly α-hydroxycarboxylic acid;
(7) the composition described in the aforementioned (1) or (2), wherein the biodegradable polymer having two or more carboxylic groups at its end is lactic acid/glycolic acid copolymer having an α,α-dicarboxylic group or an α,β,β'-tricarboxylic group at its end;
(8) the composition described in the aforementioned (1) or (2), wherein the biodegradable polymer having two or more carboxylic groups at its end is lactic acid/glycolic acid copolymer whose ω residue is tartronic acid or citric acid;
(9) the composition described in the aforementioned (1) or (2), wherein the biodegradable polymer having two or more carboxylic groups at its end is polylactic acid whose ω residue is tartronic acid or citric acid;
(10) the composition described in the aforementioned (1) or (2), wherein the physiologically active non-peptide substance is water-insoluble or slightly water-soluble;
(11) the composition described in the aforementioned (10), wherein the composition is a sustained-release composition;
(12) the composition described in the aforementioned (11), wherein the molecular weight of the physiologically active non-peptide substance is less than about 1000;
(13) the composition described in the aforementioned (11), wherein the physiologically active non-peptide substance is a gonadotropin releasing hormone agonist or antagonist;
(14) the composition described in the aforementioned (11), wherein the physiologically active non-peptide substance is a compound having a partial structure represented by the formula: 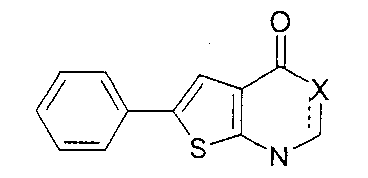 [wherein, X represents a carbon atom or a nitrogen atom, and - - - represents a single bond or a double bond] or a salt thereof [hereinafter, also abbreviated as "compound (A)"];
(15) the composition described in the aforementioned (14), wherein compound (A) is a compound represented by the formula: 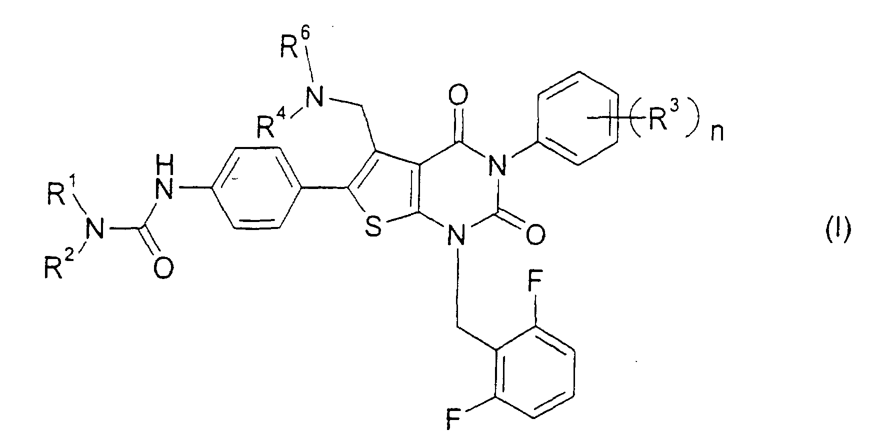 [wherein, R¹ and R² each represent a hydrogen atom, a hydroxy group, a C₁₋₄ alkoxy group, a C₁₋₄ alkoxy-carbonyl group or a C₁₋₄ alkoxy group which may have a substituent,
   R³ represents a hydrogen atom, a halogen atom, a hydroxy group or a C₁₋₄ alkoxy group which may have a substituent, or
   adjacent two R³s may be linked to form a C₁₋₄ alkylenedioxy group;
   R⁴ represents a hydrogen atom or a C₁₋₄ alkyl group;
   R⁶ represents a C₁₋₄ alkyl group which may have a substituent or a group represented by the formula: 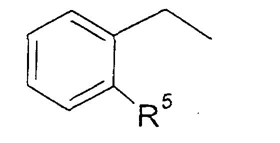 (wherein, R⁵ represents a hydrogen atom or R⁴ and R⁵ may be linked to form a heterocycle); and
   n represents an integer of 0 to 5] or a salt thereof [hereinafter also abbreviated as "compound (I)"];
(16) the composition described in the aforementioned (14), wherein compound (A) is 5-(N-benzyl-N-methylaminomethyl)-1-(2,6-difluorobenzyl)-6-[4-(3-methoxyureido)phenyl]-3-phenylthieno [2,3-d] pyrimidine-2,4(1H,3H)-dione or a salt thereof;
(17) the compound described in the aforementioned (14), wherein compound (A) is a compound represented by the formula: 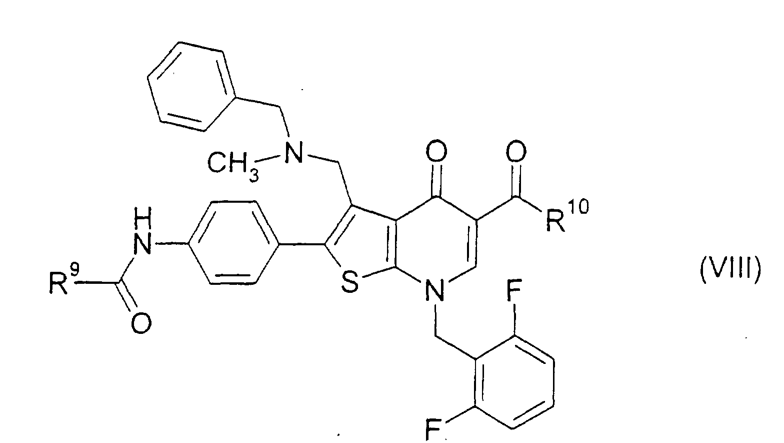 [wherein, R⁹ represents an optionally substituted C₁₋₇ alkyl group, an optionally substituted C₃₋₇ cycloalkyl group, an optionally substituted C₁₋₆ alkoxyamino group or an optionally substituted hydroxyamino group, and
   R¹⁰ represents an optionally substituted C₁₋₇ alkyl group or an optionally substituted phenyl group, respectively; or
   when R⁹ is an unsubstituted C₁₋₇ alkyl group, R¹⁰ represents a substituted C₁₋₇ alkyl group or a substituted phenyl] or a salt thereof [hereinafter, also abbreviated as "compound (VIII)"];
(18) the compound described in the aforementioned (14), wherein compound (A) is 3-(N-benzyl-N-methylaminomethyl)-4,7-dihydro-5-isobutyryl-7-(2,6-difluorobenzyl)-2-[4-[(1-hydroxycyclopropyl)carbonylamino]phenyl]-4-oxothieno[2,3-b]pyridine or a salt thereof;
(19) the composition described in the aforementioned (1) or (2), which is used for injection;
(20) the composition described in the aforementioned (1) or (2), which is in the form of a sustained-release microcapsule;
(21) a method for producing a composition characterized by blending a physiologically active non-peptide substance and a biodegradable polymer having two or more carboxylic groups at its end, or a salt thereof; and so on.

The "physiologically active non-peptide substance" used in the present invention, in particular, "water-insoluble or slightly water-soluble physiologically active non-peptide substance" may be in a free form or in a salt. Examples of such "salt" include metallic salts, ammonium salts, salts with organic bases, salts with inorganic acids, salts with organic acids, salts with basic or acidic amino acids and so on. Preferred metallic salts include alkaline metal salts such as sodium salts and potassium salts; alkaline earth metal salts such as calcium salts, magnesium salts and barium salts; aluminum salts and so on. Preferred examples of the salts with organic bases include trimethylamine, triethylamine, pyridine, picoline, ethanolamine, diethanolamine, triethanolamine, dicyclohexylamine, N,N-dibenzylethylenediamine and so on. Preferred examples of the salts with inorganic acids include salts with hydrochloric acid, hydrobromic acid, nitric acid, sulfuric acid, phosphoric acid and so on. Preferred examples of the salts with organic acids include salts with formic acid, acetic acid, trifluoroacetic acid, fumaric acid, oxalic acid, tartaric acid, maleic acid, citric acid, succinic acid, malic acid, methanesulfonic acid, benzenesulfonic acid, p-toluenesulfonic acid and so on. Preferred examples of the salts with basic amino acids include salts with arginine, lysine, ornithine and so on, and preferred examples of the salts with acidic amino acids include salts with aspartic acid, glutamic acid and so on.

Such "physiologically active non-peptide substance", in particular, "water-insoluble or slightly water-soluble physiologically active non-peptide substance" is not particularly restricted insofar as it is pharmaceutically useful, and the preferred is a synthetic organic compound. Examples of such "synthetic organic compound" include compounds including a hydrophilic part having mainly a tertiary amine, and a hydrophobic part such as chain or cyclic alkyl or aromatic group, and salts thereof. Concrete examples include cationic amphiphilic drugs (See, for example, CAD (cationic amphiphilic drug); "Pharmacological Reviews" 42(4), pp.327-354 (1990)).

Other examples of such "physiologically active non-peptide substance", in particular "water-insoluble or slightly water-soluble physiologically active non-peptide substance" include substances having a receptor agonistic or antagonistic activity, an enzyme inhibiting activity, carrier promoting or inhibiting activity and the like.

Receptors against which such "physiologically active non-peptide substance", in particular "water-insoluble or slightly water-soluble physiologically active non-peptide substance" exhibits an agonistic or antagonistic activity may exist on cellar surfaces or inside cells. Cellular surface receptors are classified into ion-channel coupled type, G-protein coupled type and enzyme coupled type. As types of ligand for such receptors, small peptide, protein, amino acid, nucleotide, steroid, fatty acid derivative, nitrogen oxide, carbon oxide and the like can be exemplified. Examples of such receptors include luteal hormone release hormone (LH-RH) receptor, thyrotropin-releasing hormone (TRH) receptor, corticotropin releasing factor (CRF) receptor, endorphin receptor, substance P receptor, neurotensin receptor, thyroid stimulating hormone (TSH) receptor, prolactin (PRL) receptor, follicle stimulating hormone (FSH) receptor, luteinizing hormone (LH) receptor, adrenocorticotropic hormone (ACTH) receptor and so on.

As enzymes against which such "physiologically active non-peptide substance", in particular "water-insoluble or slightly water-soluble physiologically active non-peptide substance" exhibits inhibiting activity, enzymes involved in the blood coagulation system, enzymes involved in the fibrinolytic system, digestive enzymes, phosphorylation enzymes, metabolizing enzymes, antioxidative enzymes and the like are exemplified. Examples of such enzymes include monoamine oxidase (MAO), angiotensin converting enzyme, HMG-CoA reductase, cholesterol esterase (ACAT), cyclooxygenase (COX), trypsin, α-chymotrypsin, kallikrein, β-galactosidase, elastase, thrombomodulin, thrombin, bloodclotting factors (I factor to X factors), protein C, protein S, plasmin, plasminogen activator, urokinase, protein kinase C, tyrosine kinase, cytochrome p450 family (3A4, 1A, 2C, 2D and so on), super oxide dismutase (SOD) and the like. As enzymes against which such CADs exhibit an inhibiting activity, those derived from human cells, bacteria, phages or viruses can be exemplified. Such CADs having an inhibiting activity are expected to have an antibacterial or antivirus activity. Examples of such enzymes include bridge-forming enzyme, transpeptidase, penicillin binding proteins (PBP-1A, PBP-1B, PBP-2, PBP-3, PBP-4, PBP-5 and PBP-6), neuraminidase, aminopeptidase A, aminopeptidase B, α-amylase, β-lactamase, reverse transcriptase and the like.

As carriers against which such "physiologically active non-peptide substance", in particular "water insoluble or slightly water-soluble physiologically active non-peptide substance" exhibits promotion or inhibition, active or passive ion channels, glucose transporters, peptide transporters, p-glycoproteins and the like are exemplified. Examples of such carriers include voltage-dependent sodium channel, calcium-dependent sodium channel, potassium-dependent calcium channel, potassium channel, chloro ion channel, gastric mucosa proton pump (H⁺, K⁺-ATPase), glucose transporters (GLUT1, GLUT2, GLUT3, GLUT4), PEPT1, MDR1, MDR2, MRP, cMOAT, ACT1 and the like.

Such "physiologically active non-peptide substance", in particular "water-insoluble or slightly water-soluble physiologically active non-peptide substance" is not particularly limited insofar as it has the above-mentioned activities, and examples of which include: antipyretic, analgesic and anti-inflammatory agents (e.g., sulpyrine, indomethacin, atropine, scopolamine, morphine, pethidine or salts thereof), tranquilizer (e.g., diazepam, lorazepam, etc.), antimicrobials (e.g., griseofulvin, etc.), antibiotics (e.g., dibekacin, Kanendomycin, lividomycin, tobramycin, amikacin, fradiomycin, sisomicin, tetracycline, oxytetracycline, rolitetracycline, doxycycline, ampicillin, moxalactam, thienamycin, sulfazecin, aztreonam or salts thereof etc.), antitumor agents (e.g., fumagillin, mitomycin C, adriamycin, fluorouracil, etc.), cholesterol-lowering agents (e.g., clofibrate), antitussive expectorants (e.g., ephedrine, methylephedrine, noscapine, codeine, dihydrocodeine, chloperastine, protokylol, isoproterenol, salbutamol, terbutaline or salts thereof etc.), muscle relaxants (e.g., pridinol, pancuronium, etc.), antiepileptics (e.g., acetazolamide, chlordiazepoxide, etc.), antiulcer agents (e.g., metoclopramide, etc.), antidepressants (e.g., clomipramine, etc.), anti-allergic agents (diphenhydramine, tripelenuamine, diphenylpyraline, methoxyphenamine, etc.), cardiotonics (e.g., etilefrine, etc.), antiarrhythmic agents (e.g., alprenolol, bufetolol, oxprenolol, etc.), vasodilators (e.g., oxyfedrine, bamethan, etc.), hypotensive diuretics (e.g., pentolinium, mecamylamine, clonidine, etc.), antidiabetics (e.g., glybuzole, etc.), antituberculous agents (e.g., ethambutol, etc.), narcotic antagonists (e.g., levallorphan, nalorphine, naloxone, or salts thereof etc.) and hormones (e.g., estrogen, luteinizing hormones (LHs), dexamethasone, hexestrol, betamethasone, triamcinolone, triamcinolone acetonide, fluocinolone acetonide, predonizolone, hydrocortisone, etc.), as well as lipid soluble vitamins (e.g., vitamin A, vitamin D, vitamin E, vitamin K, folic acid (vitamin M), etc.).

Examples where the "physiologically active non-peptide substance", in particular "water-insoluble or slightly water-soluble physiologically active non-peptide substance" has a property as a CAD include, those exhibiting receptor antagonistic activity (e.g., amiodarone, promethazine, propranolol, etc.), those exhibiting enzyme inhibiting activity (e.g., chloramphenicol, gentamicin, etc.), and those exhibiting carrier antagonistic activity (e.g., amitriptyline, imipramine, trimipramine, etc.).

Preferably, such "physiologically active non-peptide substance", in particular "water-insoluble or slightly water-soluble physiologically active non-peptide substance" has a molecular weight of less than about 1000, preferably less than about 900, more preferably less than about 800, and most preferably less than about 700.

Such "water-insoluble or slightly water-soluble physiologically active non-peptide substance" has a solubility of, for example, less than 0.1% (w/w), preferably less than 0.01% (w/v). The term "solubility" used herein means concentration of drug in a supernatant which is obtained by centrifugal separation of unsolved agent, after shaking a mixture prepared by adding the agent to the second solution defined in Japanese Pharmacopoeia [0.2 M phosphate-buffer (pH approx. 6.8), for more than 30 minutes at room temperature (about 15 to about 25°C) in a rate of more than 100 times per minutes using, for example, a Recipro shaker (model SR-I, Taiyo Scientific Industrial Co., Ltd.).

As such "physiologically active non-peptide substance", in particular "water-insoluble or slightly water-soluble physiologically active non-peptide substance", gonadotropin releasing hormone (GnRH) agonist or antagonist is preferred, and GnRH antagonist is more preferred. The GnRH has a pseudo LH-RH (Luteinizing hormone-releasing hormone) activity.

As such "GnRH antagonist", any compounds are possible insofar as they have a GnRH antagonistic activity, and examples of which include compounds having a partial structure (basic structure) represented by the formula: 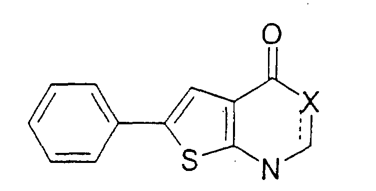 [wherein, X represents a carbon atom or a nitrogen atom, - - - represents a single bond or a double bond] or salts thereof, and more specifically the aforementioned compound (I), compound (VIII) and the like are recited.

Definitions for the respective substituents in the above formula (I) are as follows.

Examples of "C₁₋₄ alkoxy group" represented by R¹ or R² include methoxy, ethoxy, propoxy, isopropoxy, butoxy, tert-butoxy and the like. Among these, C₁₋₃ alkoxy group is preferred. Methoxy is more preferred.

Examples of "C₁₋₄ alkoxy-carbonyl group" represented by R¹ or R² include methoxycarbonyl, ethoxycarbonyl, propoxycarbonyl, isopropoxycarbonyl, butoxycarbonyl, tert-butoxycarbonyl and the like. Among these C₁₋₃ alkoxy-carbonyl group is preferred. Methoxycarbonyl is more preferred.

Examples of the "C₁₋₄ alkyl group" in the "optionally substituted C₁₋₄ alkyl group" represented by R¹ or R² include linear C₁₋₄ alkyl groups (e.g., methyl, ethyl, propyl, butyl, etc.) and branched C₃₋₄ alkyl groups (e.g., isopropyl, isobutyl, sec-butyl, tert-butyl, etc.). Among these, C₁₋₃ alkyl groups are preferred. In particular, ethyl is preferred.

Examples of substituent in "optionally substituted C₁₋₄ alkyl group" represented by R¹ or R² include (i) hydroxy, (ii) C₁₋₇ acyloxy (e.g., C₁₋₆ alkyl-carbonyloxy such as acetoxy and propionyloxy), (iii) benzoyloxy, (iv) amino groups which may have one or two substituent(s) selected from the group consisting of C₁₋₆ alkoxy-carbonyl (e.g., methoxycarbonyl, ethoxycarbonyl, tert-butoxycarbonyl, etc.), benzyloxycarbonyl, C₁₋₄ acyl (e.g., C₁₋₃ alkyl-carbonyl such as acetyl and propionyl), C₁₋₄ alkyl (e.g., methyl, ethyl, propyl, butyl, etc.), C₁₋₃ alkylsulfonyl (e.g., methanesulfonyl) and the like (for example, amino, dimethylamino, methoxycarbonylamino, ethoxycarbonylamino, tert-butoxycarbonylamino, benzyloxycarbonylamino, acetylamino, methanesulfonylamino and the like), (v) C₁₋₁₀ alkoxy (e.g., methoxy, ethoxy, propoxy, tert-butoxy, etc.), (vi) C₃₋₇ cycloalkyloxycarbonyloxy-C₁₋₃ alkoxy (e.g., cyclohexyloxycarbonyloxy-1-ethoxy, etc.) and (vii) C₁₋₃ alkoxy-C₁₋₃ alkoxy (e.g., methoxymethoxy, methoxyethoxy, etc.). Among these, hydroxy is preferred.

The "C₁₋₄ alkyl group" in the "optionally substituted C₁₋₄ alkyl group" represented by R¹ or R² may have 1 to 5 substituent(s), preferably 1 to 3 substituent(s) at positions where substitution is possible, and if the number of substitution is two or more, the substituents may be the same or different.

It is preferred that one of R¹ and R² is a hydrogen atom, while the other of R¹ and R² is C₁₋₃ alkoxy group.

Examples of the "halogen atom" represented by R³ include fluorine, chlorine, bromine and iodine. Among these, chlorine is preferred.

Examples of the "C₁₋₄ alkoxy group" in the "optionally substituted C₁₋₄ alkoxy group" represented by R³ include methoxy, ethoxy, propoxy, isopropoxy, butoxy, tert-butoxy and the like. Among these, methoxy is preferred.

Examples of "substituent(s)" in "optionally substituted C₁₋₄ alkoxy group" represented by R³ include those as same as recited for the "substituent" in "optionally substituted C₁₋₄ alkyl group" represented by R¹ or R². Among these, C₁₋₄ alkoxy group is preferred.

The C₁₋₄ alkoxy group may have 1 to 5 substituent(s), preferably 1 to 3 substituent(s) at positions where substitution is possible, and if the number of substitution is two or more, the substituents may be the same or different.

Examples of "C₁₋₄ alkylenedioxy group" which is formed by linked adjacent two R³ include methylenedioxy, ethylenedioxy and the like.

Preferably, R³ is a hydrogen atom.

Examples of the "C₁₋₄ alkyl group" represented by R⁴ include linear C₁₋₄ alkyl groups (e.g., methyl, ethyl, propyl, butyl, etc.), branched C₃₋₄ alkyl groups (e.g., isopropyl, isobutyl, sec-butyl, tert-butyl, etc.) and the like. Among these, C₁₋₃ alkyl groups are preferred. In particular, methyl is preferred.

Examples of the "optionally substituted C₁₋₄ alkyl group" represented by R⁶ include "optionally substituted C₁₋₄ alkyl groups" represented by R¹ or R².

Examples of the "heterocycle" formed by linkage of R⁴ and R⁵ include 5- or 6-membered nitrogen-containing heterocyclic groups. When R⁴ and R⁵ are linked to each other, examples of groups represented by the formula: 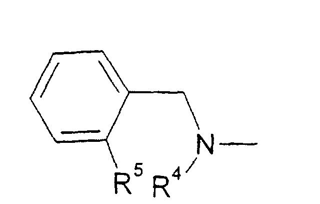 include the groups represented by the formulas 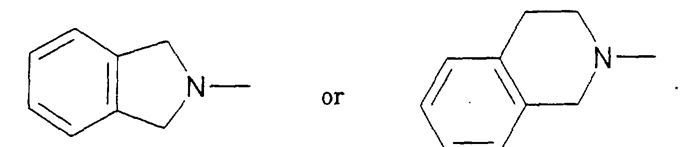 Among these, group represented by the formula: 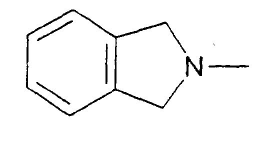 is preferred.

Preferably, R⁶ is a group represented by the following formula: 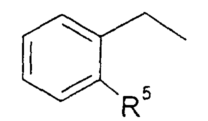 [wherein, R⁵ has the same meaning as described above].

Preferably, R⁴ is a C₁₋₃ alkyl group and R⁵ is a hydrogen atom.

Preferably, n is an integer of 0 to 2.

Examples of preferred compounds in compound (I) include those wherein R¹ is hydroxy group, methoxy group or C₁₋₃ alkyl group; R² is a hydrogen atom or a C₁₋₃ alkyl group; R⁴ is C₁₋₃ alkyl group; R⁶ is a benzyl group; and n is 0, and salts thereof.

Among others, compounds wherein R¹ represents a methoxy group, R² and R⁵ each represent a hydrogen atom; R⁴ is a C₁₋₃ alkyl group; R⁶ is a benzyl group; and n is 0, and salts thereof are recited.

Concrete examples of compound (I) include 5-(N-benzyl-N-methylaminomethyl)-1-(2,6-difluorobenzyl)-6-[4-(3-methoxyureido)phenyl]-3-phenylthieno[2,3-d]pyrimidine-2,4(1H,3H)-dione,
5-(N-benzyl-N-methylaminomethyl)-1-(2,6-difluorobenzyl)-6-[4-(3-hydroxyureido)phenyl]-3-phenylthieno[2,3-d]pyrimidine-2,4(1H,3H)-dione,
5-(N-benzyl-N-methylaminomethyl)-1-(2,6-difluorobenzyl)-6-[4-(3-methylureido)phenyl]-3-phenylthieno[2,3-d]pyrimidine-2,4(1H,3H)-dione,
5-(N-benzyl-N-methylaminomethyl)-1-(2,6-difluorobenzyl)-6-[4-(3-ethylureido)phenyl]-3-phenylthieno[2,3-d]pyrimidine-2,4(1H,3H)-dione and salts thereof are exemplified.

Among them, 5-(N-benzyl-N-methylaminomethyl)-1-(2,6-difluorobenzyl)-6-[4-(3-methoxyureido)phenyl]-3-phenylthieno[2,3-d]pyrimidine-2,4(1H,3H)-dione or a salt thereof is preferred.

Definition of each substituent in the above formula (VIII) will be described below.

Examples of the "C₁₋₇ alkyl group" in the "optionally substituted C₁₋₇ alkyl group" represented by R⁹ include linear C₁₋₇ alkyl groups (e.g., methyl, ethyl, propyl, butyl, pentyl, hexyl, heptyl, etc.) and branched C₃₋₇ alkyl groups (e.g., isopropyl, isobutyl, sec-butyl, tert-butyl, isopentyl, neopentyl, etc.). Among these, branched C₃₋₇ alkyl groups are preferred. In particular, isopropyl is preferred.

Examples of the "substituents" in the "optionally substituted C₁₋₇ alkyl group" represented by R⁹ include (i) hydroxy, (ii) C₁₋₇ acyloxy (e.g., C₁₋₆ alkyl-carbonyloxy such as acetoxy and propionyloxy; benzoyloxy, etc.), (iii) amino groups which may have one or two substituent(s) selected from the group consisting of C₁₋₆ alkoxy-carbonyl (e.g., methoxycarbonyl, ethoxycarbonyl, tert-butoxycarbonyl, etc.), benzyloxycarbonyl, C₁₋₃ acyl (e.g., C₁₋₂ alkyl-carbonyl such as acetyl and propionyl, etc.), C₁₋₃ alkylsulfonyl (e.g., methanesulfonyl, etc.), C₁₋₃ alkyl (e.g., methyl, ethyl, etc.) and the like (for example, amino, methoxycarbonylamino, ethoxycarbonylamino, tert-butoxycarbonylamino, benzyloxycarbonylamino, acetylamino, methanesulfonylamino, methylamino, dimethylamino and the like), (iv) C₁₋₁₀ (preferably C₁₋₄) alkoxy which may have 1 to 3 substituent(s) selected from the group consisting of C₃₋₇ cycloalkyloxycarbonyloxy (e.g., cyclohexyloxycarobonyloxy, etc.) and C₁₋₃ alkoxy (e.g., methoxy, ethoxy, etc.) (for example, methoxy, ethoxy, propoxy, tert-butoxy, cyclohexyloxycarbonyloxy-1-ethoxy, methoxymethoxy, ethoxymethoxy, etc.), (v) C₁₋₆ alkoxy-carbonyl (e.g., methoxycarbonyl, ethoxycarbonyl, propoxycarbonyl, etc.) and the like. Among these, hydroxy group is preferred.

Such "C₁₋₇ alkyl group" may have 1 to 5 substituent(s), preferably 1 to 3 substituent(s) at positions where substitution is possible, and if the number of substitution is two or more, the substituents may be the same or different.

Examples of the "C₃₋₇ cycloalkyl group" in the "optionally substituted C₃₋₇ cycloalkyl group" represented by R⁹ include cyclopropyl, cyclobutyl, cyclopentyl, cyclohexyl, cycloheptyl and the like. Among these, cyclopropyl is preferred.

As the "substituent(s)" in the "optionally substituted C₃₋₇ cycloalkyl group" represented by R⁹, 1 to 3 substituent(s) as same as those in the "substituent(s)" in the "optionally substituted C₁₋₇ alkyl group" represented by R⁹ can be recited. When the number of substituent is two or more, the substituents may be the same or different to each other.

Examples of the "C₁₋₆ alkoxyamino group" in the "optionally substituted C₁₋₆ alkoxyamino group" represented by R⁹ include mono- or di-C₁₋₆ alkoxyamino groups (e.g., methoxyamino, ethoxyamino, dimethoxyamino, diethoxyamino, ethoxymethoxyamino, etc.). Among these, mono-C₁₋₃ alkoxyamino groups (e.g., methoxyamino, etc.) are preferred.

As the "substituent(s)" in the "optionally substituted C₁₋₆ alkoxyamino group" represented by R⁹, the same number and the same kinds of substituent(s) as those in the "substituent(s)" in the "optionally substituted C₁₋₇ alkyl group" represented by R⁹ can be recited. When the number of substituent is two or more, the substituents may be the same or different to each other. Such "substituent(s)" may substitute for "C₁₋₆ alkoxy group" or "amino group" of C₁₋₆ alkoxyamino group.

Concrete examples of such "optionally substituted C₁₋₆ alkoxyamino group" include methoxyamino, N-methyl-N-methoxyamino, N-ethyl-N-methoxyamino, ethoxyamino, dimethoxyamino, diethoxyamino, ethoxymethoxyamino, and the like. Preferred examples include C₁₋₃ alkoxyamino groups, N-C₁₋₃ alkyl-N-C₁₋₃ alkoxyamino groups, and the like.

The "substituent(s)" in the "optionally substituted hydroxyamino group" represented by R⁹ may substitute for "hydroxy group" or "amino group" in a hydroxyamino group, and examples of the substituent on the "hydroxy group" include (i) C₁₋₇ acyl groups (e.g., C₁₋₆ alkyl-carbonyl such as acetyl and propionyl; benzoyl, etc.), (ii) amino groups which may have one or two substituent(s) selected from the group consisting of C₁₋₆ alkoxy-carbonyl (e.g., methoxycarbonyl, ethoxycarbonyl, tert-butoxycarbonyl, etc.), benzyloxycarbonyl, C₁₋₃ acyl (e.g., C₁₋₂ alkyl-carbonyl such as acetyl and propionyl), C₁₋₃ alkylsulfonyl (e.g., methanesulfonyl, etc.) and C₁₋₃ alkyl (e.g., methyl, ethyl, etc.) (for example, amino, methoxycarbonylamino, ethoxycarbonylamino, tert-butoxycarbonylamino, benzyloxycarbonylamino, acetylamino, methanesulfonylamino, methylamino, dimethylamino, etc.), (iii) C₁₋₁₀ (preferably C₁₋₄) alkyl groups which may have one to three substituent(s) selected from the group consisting of C₃₋₇ cycloalkyloxycarbonyloxy (e.g., cyclohexyloxycarbonyloxy, etc.) and C₁₋₃ alkoxy (e.g., methoxy, ethoxy, etc.) (for example, methyl, ethyl, propyl, tert-butyl, cyclohexyloxycarbonyloxy-1-ethyl, methoxymethyl, ethoxymethyl, etc.), and examples of the substituent(s) on the "amino group" include groups described in the above (i) to (iii). The substituents "hydroxy group" and "amino group" on the hydroxyamino group may be the same or different with each other.

Preferred examples of the "optionally substituted hydroxyamino group" include N-C₁₋₆ alkyl-N-hydroxyamino groups (e.g., N-methyl-N-hydroxyamino, N-ethyl-N-hydroxyamino and the like). More preferred examples include N-C₁₋₃ alkyl-N-hydroxyamino groups.

Examples of the "C₁₋₇ alkyl group" in the "optionally substituted C₁₋₇ alkyl group" represented by R¹⁰ include linear or branched C₁₋₇ alkyl groups (e.g., methyl, ethyl, propyl, isopropyl, butyl, isobutyl, sec-butyl, tert-butyl, pentyl, isopentyl, neopentyl, hexyl, heptyl, etc.). Among these, C₁₋₃ alkyl groups (e.g., methyl, ethyl, propyl, isopropyl, etc.) are preferred. Isopropyl is particularly preferred.

As the "substituent(s)" in the "optionally substituted C₁₋₇ alkyl group" represented by R¹⁰, the same number and the same kinds of substituent(s) as those in the "substituent(s)" in the "optionally substituted C₁₋₇ alkyl group" represented by R⁹ can be recited. When the number of substituent is two or more, the substituents may be the same or different to each other.

Examples of the "substituent(s)" in the "optionally substituted phenyl group" represented by R¹⁰ include halogens (e.g. fluorine, chlorine, bromine, iodine, etc.), C₁₋₃ alkyl groups (e.g., methyl, ethyl, propyl, isopropyl, etc.), C₁₋₃ alkoxy groups (e.g., methoxy, ethoxy, propoxy, isopropoxy, etc.). Among these halogens (preferably fluorine) are preferred.

Such "phenyl group" may have 1 to 5 substituent(s). preferably 1 to 3 substituent(s) at positions where substitution is possible, and if the number of substituent is two or more, the substituents may be the same or different.

R⁹ is preferably a substituted branch C₃₋₇ alkyl group or a substituted C₃₋₇ cycloalkyl group, more preferably a branch C₃₋₇ alkyl group substituted by a hydroxy group or a C₃₋₇ cycloalkyl group substituted by a hydroxy group. Among these, C₃₋₇ cycloalkyl groups substituted by a hydroxy group are preferred. Also C₁₋₃ alkyl groups which may be substituted by a hydroxy group, C₃₋₇ cycloalkyl groups which may be substituted by a hydroxy group, as well as mono-C₁₋₃ alkoxyamino groups, N-C₁₋₃ alkyl-N-hydroxyamino groups, hydroxyamino group and the like are preferred. Especially preferred R⁹ is a methoxyamino group or a cyclopropyl group which may be substituted by a hydroxy group. A cyclopropyl group substituted by a hydroxy group is most preferred.

Preferably, R¹⁰ is an optionally substituted C₁₋₇ alkyl group. More preferably, R¹⁰ is a C₁₋₃ alkyl group which may be substituted by a hydroxy group or the like. Especially preferred R¹⁰ is isopropyl. Also phenyl is preferred.

Preferred examples of compound (VIII) are compounds wherein R⁹ is a C₁₋₃ alkyl group which may be substituted by a hydroxy group, a C₃₋₇ cycloalkyl group which may be substituted by a hydroxy group or a mono-C₁₋₃ alkoxyamino group; and R¹⁰ is a C₁₋₃ alkyl group or a phenyl group, and salts thereof.

As more preferably compounds, compounds wherein R⁹ is (1) a C₁₋₃ alkyl group substituted by one or two hydroxy group(s), (2) a C₃₋₇ cycloalkyl group substituted by a hydroxy group, or (3) a C₁₋₃ alkoxyamino group; and R¹⁰ is an isopropyl or phenyl, and salts thereof.

Concrete examples of compound (VIII) include 3-(N-benzyl-N-methylaminomethyl)-4,7-dihydro-5-isobutyryl-7-(2,6-difluorobenzyl)-2-(4-cyclopropanecarbonylaminophenyl)-4-oxothieno[2,3-b]pyridine,
5-benzoyl-3-(N-benzyl-N-methylaminomethyl)-7-(2,6-difluorobenzyl)-4,7-dihydro-4-oxo-2-[4-(3-hydroxy-2-methylpropionylamino)phenyl]thieno[2,3-b]pyridine,
5-(4-fluorobenzoyl)-3-(N-benzyl-N-methylaminomethyl)-7-(2,6-difluorobenzyl)-4,7-dihydro-4-oxo-2-(4-cyclopropane carbonylaminophenyl)thieno[2,3-b]pyridine,
3-(N-benzyl-N-methylaminomethyl)-4,7-dihydro-5-isobutyryl-7-(2,6-difluorobenzyl)-2-[4-(3-hydroxy-2-methylpropionylamino)phenyl]-4-oxothieno[2,3-b]pyridine,
3-(N-benzyl-N-methylaminomethyl)-4,7-dihydro-5-isobutyryl-7-(2,6-difluorobenzyl)-2-(4-N'-methoxyureidophenyl)-4-oxothieno[2,3-b]pyridine,
3-(N-benzyl-N-methylaminomethyl)-4,7-dihydro-5-isobutyryl-7-(2,6-difluorobenzyl)-2-[4-[(1-hydroxycyclopropyl)carbonylamino]phenyl]-4-oxothieno[2,3-b]pyridine,
(R)-4,7-dihydro-2-[4-(3-hydroxy-2-methylpropyonylamino)phenyl]-7-(2,6-difluorobenzyl)-3-(N-benzyl-N-methylaminomethyl)-5-isobutyryl-4-oxothieno[2,3-b]pyridine,
4,7-dihydro-2-[4-(2-hydroxy-2-methylpropyonylamino)phenyl]-7-(2,6-difluorobenzyl)-3-(N-benzyl-N-methylaminomethyl)-5-isobutyryl-4-oxothieno[2,3-b]pyridine,
4,7-dihydro-2-[4-(3-hydroxy-3-methylbutyrylamino)phenyl]-7-(2,6-difluorobenzyl)-3-(N-benzyl-N-methylaminomethyl)-5-isobutyryl-4-oxothieno[2,3-b]pyridine,
(R)-4,7-dihydro-2-[4-(2,3-dihydroxypropionylamino)phenyl]-7-(2,6-difluorobenzyl)-3-(N-benzyl-N-methylaminomethyl)-5-isobutyryl-4-oxothieno[2,3-b]pyridine,
3-(N-benzyl-N-methylaminomethyl)-5-benzoyl-7-(2,6-difluorobenzyl)-4,7-dihydro-2-[4-[(1-hydroxycyclopropyl)carbonylamino]phenyl]-4-oxothieno[2,3-b]pyridine or salts thereof.

Among them, 3-(N-benzyl-N-methylaminomethyl)-4,7-dihydro-5-isobutyryl-7-(2,6-difluorobenzyl)-2-[4-[(1-hydroxycyclopropyl)carbonylamino]phenyl]-4-oxothieno[2,3-b]pyridine or a salt thereof is preferred.

As salts of compound (I) and compound (III), physiologically acceptable acid addition salts are preferred. Examples of such salts include salts with inorganic acids (e.g., hydrochloric acid, hydrobromic acid, nitric acid, sulfuric acid, phosphoric acid, etc.), and salts with organic acids (e.g., formic acid, acetic acid, trifluoroacetic acid, fumaric acid, oxalic acid, tartaric acid, maleic acid, citric acid, succinic acid, malic acid, methanesulfonic acid, benzenesulfonic acid, p-toluenesulfonic acid, etc.). When compound (I) has an acidic group, physiologically acceptable salts may be formed together with inorganic bases (alkaline metal salts or alkaline earth metals such as sodium, potassium, calcium, magnesium and the like, and ammonia and the like), or organic bases (e.g., trimethylamine, triethylamine, pyridine, picoline, ethanolamine, diethanolamine, triethanolamine, dicyclohexylamine, N,N'-dibenzylethylenediamine, etc.).

Compound (I) can be produced in accordance with a per se known method as disclosed, for example, in JP 9-169768 or WO 96/24597 or analogous methods thereto. As concrete examples, Production method 1 and Production method 2 described below can be recited. Compounds in any formulas may form salts, and examples of such salts include those as same as salts of compound (I).

### (Production Method 1)

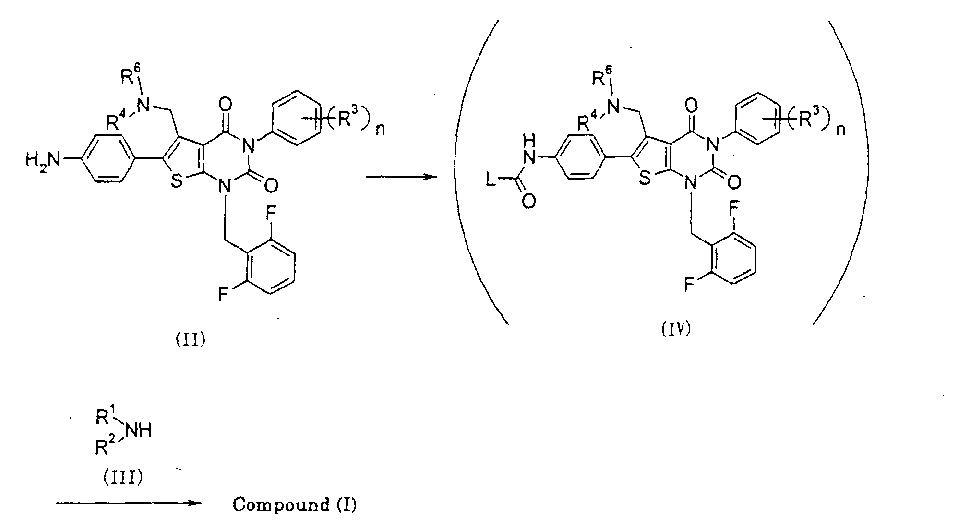

In the above formulae, L represents a leaving group, and other symbols are as defined above.

The "leaving group" for L includes, for example, 1-imidazolyl, a halogen atom, an alkoxy group which may be substituted, etc. The "alkoxy group which may be substituted" includes, for example, C₁₋₄ alkoxy groups which may be substituted by 1 to 3 halogen atom(s) such as chlorine, bromine, etc. (e.g., 2,2,2-trichloroethoxy group, etc.).

Compound (II) can be produced by the methods as disclosed in JP 9-169768 or analogous methods thereto.

Compound (I) can be produced by reacting compound (II) with carbonyldiimidazole (N,N'-carbonyldiimidazole; CDI) or phosgene (including dimer and trimer) to obtain compound (IV), followed by reacting with compound (III). The reaction can be carried out without isolation of compound (IV), or compound (IV) can be used as an isolated form in the next reaction.

Compound (IV) can also be produced by reacting compound (II) with, for example, a chloroformic acid ester compound (e.g., 2,2,2-trichloroethyl chloroformate, 1-chloroethyl chloroformate, etc.).

In the reaction of compound (II) with carbonyldiimidazole or phosgene, etc., carbonyldiimidazole or phosgene, etc. is used in amount of about 1 to 3 moles, relative to one mole of compound (II).

This reaction is advantageously carried out in a solvent which will not adversely affect on the reaction.

Examples of such solvent include ethers (e.g., ethyl ether, dioxane, dimethoxyethane, tetrahydrofuran, etc.), aromatic hydrocarbons (e.g., benzene, toluene, etc.), amides (e.g., dimethylformamide, dimethylacetamide, etc.), halogenated hydrocarbons (e.g., chloroform, dichloromethane, etc.), and so on.

The reaction temperature is usually about 0 to 150°C, preferably room temperature (about 15 to about 25°C). The reaction time is usually about 1 to about 36 hours.

This reaction is carried out in the presence of a base if necessary.

The "base" is exemplified by inorganic bases such as sodium carbonate, sodium hydrogen carbonate, potassium carbonate, potassium hydrogen carbonate, sodium hydroxide, potassium hydroxide and thallium hydroxide, and organic bases such as triethylamine and pyridine, etc.

The amount of the "base" is about 2 to 20 moles, preferably about 5 to 12 moles, relative to one mole of compound (II).

The subsequent reaction with compound (III) can be carried out in the same condition as the above reaction of compound (II) with carbonyldiimidazole or phosgene. The amount of compound (III) is about 2 to 20 moles, preferably about 5 to 10 moles, relative to one mole of compound (II) or compound (IV). The reaction temperature is usually about 0 to 150°C, preferably room temperature (about 15 to 25°C). The reaction time is usually about 1 to 6 hours.

Compound (III) and carbonyldiimidazole or phosgene can be reacted with compound (II) at the same time.

### (Production Method 2)

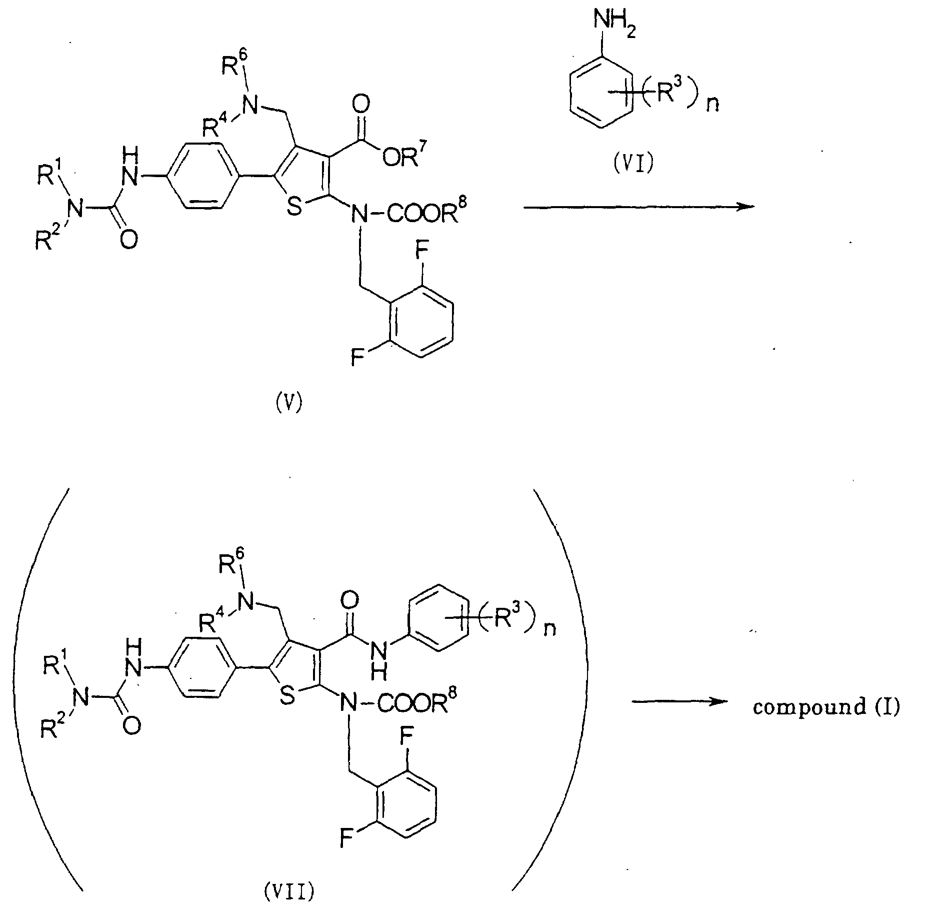

In the above formulae, R⁷ represents a hydrogen atom or an alkyl group, R⁸ represents an alkyl group, and other symbols are as defined above.

Examples of the "alkyl group" represented by R⁷ or R⁸ includes those recited for the "C₁₋₄ alkyl group" of the "optionally substituted C₁₋₄ alkyl group" represented by R¹ or R².

Compound (V) can be produced in any per se known manner, for example, p-nitrophenylacetone is reacted with a cyanoacetic acid ester compound and sulfur [e.g., Chem. Ber., 99, 94-100(1966)], and thus obtained 2-amino-4-methyl-5-(4-nitrophenyl)thiophene is subjected to the methods disclosed in JP 9-169768, WO 96/24597 or analogous methods thereto.
1) When R⁷ is a hydrogen atom, compound (I) can be produced by reacting compound (V) with a compound of the formula: 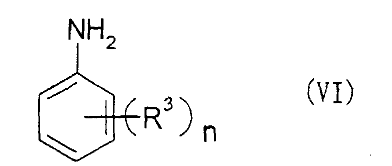 [wherein each symbol is as defined above], or a salt thereof [hereinafter, also abbreviated as compound (VI)], in the presence of a condensing agent, to obtain compound (VII), following by subjecting to cyclization.
   The "condensing agent" includes, for example, benzotriazol-1-yloxytripyrrolidinophosphonium hexafluorophosphate (PyBOP), etc.
   The amount of the "condensing agent" is about 1 to 3 moles, relative to one mole of compound (V).
   This reaction is advantageously carried out in a solvent which will not adversely affect on the reaction.
   Examples of such solvent include alcohols (e.g., ethanol, methanol, etc.), aromatic hydrocarbons (e.g., benzene, toluene, etc.), amides (e.g., dimethylformamide, dimethylacetamide, etc.), halogenated hydrocarbons (e.g., chloroform, dichloromethane, etc.), and so on.
   The reaction temperature is usually about 0 to about 150°C, preferably room temperature (about 15 to about 25°C). The reaction time is usually about 1 to about 36 hours.
   The product as produced in the manner mentioned above may be applied to the next reaction while it is still crude in the reaction mixture, or may be isolated from the reaction mixture in any ordinary manner.
   Compound (VII) is subjected to cyclization in the presence of a base.
   The "base" is exemplified by inorganic bases such as sodium methoxide, sodium carbonate, sodium hydrogen carbonate, potassium carbonate, potassium hydrogen carbonate, sodium hydroxide, potassium hydroxide and thallium hydroxide, and organic bases such as triethylamine and pyridine, etc.
   The amount of the "base" is about 2 to 20 moles, preferably about 5 to 12 moles, relative to one mole of compound (VII).
   This reaction is advantageously carried out in a solvent which will not adversely affect on the reaction.
   Examples of such solvent include alcohols (e.g., ethanol, methanol, etc.), aromatic hydrocarbons (e.g., benzene, toluene, etc.), amides (e.g., dimethylformamide, dimethylacetamide, etc.), halogenated hydrocarbons (e.g., chloroform, dichloromethane, etc.), and so on.
   The reaction temperature is usually about 0 to about 150°C, preferably room temperature (about 15 to about 25°C). The reaction time is usually about 1 to about 36 hours.
2) When R⁷ is an alkyl group, compound (I) can be produced by reacting compound (V) with an activated compound (VI).

Activated compound (VI) can be produced in any per se known manner, for example, by reacting an organoaluminum reagent with compound (VI) in a solvent inert to the reaction.

The "organoaluminum reagent" includes, for example, trimethyl aluminum, dimethyl aluminum chloride, etc, and a solution including them, etc.

The amount of the "organoaluminum reagent" is about 1 to 5 moles, preferably about one mole, relative to one mole of compound (VI).

Examples of the solvent include halogenated hydrocarbons (e.g., chloroform, dichloromethane, etc.), and so on.

The reaction temperature is usually about 0 to 150°C, preferably room temperature (about 15 to 25°C). The reaction time is usually about 1 to 6 hours.

The cyclization can be carried out by reacting compound (V) with an activated compound (VI) to obtain compound (I).

The amount of "compound (V)" is about one fifth of the amount of mixture of compound (VI) and the organoaluminum reagent.

This reaction is advantageously carried out in a solvent which will not adversely affect on the reaction.

Such a solvent is the same as those used in the reaction to obtain an activated compound (VI).

The reaction temperature is usually about 0 to 150°C, preferably room temperature (about 15 to 25°C). The reaction time is usually about 1 to 48 hours.

Compound (I) may be isolated and purified by ordinary means of separation such as recrystallization, distillation and chromatography, etc.

When compound (I) is obtained in free form, it can be converted to a salt by per se known methods or analogous thereto. When compound (I) is obtained in salt form, it can be converted to the free form or another salt by per se known methods or analogous thereto. Compound (I) may be a hydrate or a non-hydrate. The hydrate is exemplified by monohydrate, sesquihydrate and dihydrate. When compound (I) is obtained as a mixture of optically active configurations, it can be resolved into the (R)- and (S)-forms by per se known optical resolution techniques. Compound (I) may be labeled with an isotope (e.g., ³H, ¹⁴C, ³⁵S, etc.).

Compound (VIII) or a salt thereof can be produced in any per se known manner, disclosed, for example in WO 95/28405, WO 00/00493 or analogous methods thereto.

Examples of the "biodegradable polymer having two or more carboxylic groups at its end" include biodegradable polymers having two or more, preferably two or three carboxylic groups. Among these, biodegradable polymers having an α,α-dicarboxylic group or an α,β,β'-tricarboxylic group at their end are preferred.

The term "end" used herein means that of a minimum repeating unit that is bound to an adjacent minimum repeating unit at its one end, among minimum repeating units constituting the polymer. Where a main chain of polymer has a hetero atom, an end which occurs on the left side is referred to as an α residue and an end which occurs on the right side is referred to as an ω residue when minimum repeating units are represented from left side while giving a priority to the hetero atom. A linear polymer has two ends, and a tandem polymer and a star-shaped polymer which are branch polymers have two or more ends.

Examples of such "biodegradable polymer having two or more carboxylic groups at its end" include polymers whose ω residue is a hydroxypolycarboxylic acid (e.g., tartronic acid, 2-hydroxyethylmalonic acid, malic acid, citric acid, etc.), and part other than the ω residue is a aliphatic polyester [for example, polymers or copolymers synthesized from one or more kinds of α-hydroxycarboxylic acids (e.g., glycolic acid, lactic acid, 2-hydroxybutyric acid, etc.), α-hydroxydicarboxylic acids (e.g., malic acid, etc.), α-hydroxytricarboxylic acids (e.g., citric acid, etc.), and so on], poly(α-cyanoacrylic acid ester), poly amino acids [e.g., poly(γ-benzyl-L-glutamic acid), etc.], maleic anhydride based copolymers (e.g., styrene-maleic acid copolymer, etc.) and the like. Monomers may be bonded in any of random, block, graft manners. Also, where the above-mentioned α-hydroxymonocarboxylic acids, α-hydroxydicarboxylic acids, α-hydroxytricarboxylic acids have an intramolecular optical active center, any of D-, L-, DL- forms may be used. The ω residue is preferably, tartaric acid, citric acid or 2-hydroxyethylmalonic acid, and more preferably tartaric acid or citric acid. The part other than the ω residue is preferably poly α-hydroxycarboxylic acids. Among them, linear poly α-hydroxycarboxylic acids are preferred.

Examples of α-hydroxycarboxylic acid which is a minimum repeating unit of the above "poly α-hydroxycarboxylic acid" include lactic acid, glycolic acid and the like. Examples of such "poly α-hydroxycarboxylic acid" include copolymers of such as lactic acid and glycolic acid [hereinafter, also referred to as poly(lactide-co-glicolide), poly(lactic acid-co-glycolic acid) or lactic acid-glycolic acid copolymer]. The "lactic acid-glycolic acid copolymer" means homopolymers of lactic acid and glycolic acid (polymer, polylactide or polyglycolide) and copolymers thereof.

A composition ratio of lactic acid and glycolic acid (lactic acid/glycolic acid; mol/mol %) in the "lactic acid-glycolic acid copolymer" is not particularly limited insofar as the object of the present invention is achieved, and is, for example, about 100/0 to about 30/70, preferably about 100/0 to about 40/60, more preferably about 100/0 to about 45/55.

In the case where an α-hydroxycarboxylic acid which is to be a minimum repeating unit of the "poly α-hydroxycarboxylic acid" has an intramolecular optical active center, any of D-, L- and DL- forms may be used. For example, α-hydroxycarboxylic acids having a ratio of D-form/L-form (mol/mol%) of about 75/25 to about 25/75, preferably about 60 /40 to about 30/70 are used.

Preferred examples of the "biodegradable polymer having two or more carboxylic groups at its end" include lactic acid-glycolic acid copolymers having an α,α-dicarboxylic group or an α,β,β'-tricarboxylic group at their end. Lactic acid-glycolic acid copolymers whose ω residue is tartaric acid, lactic acid-glycolic acid copolymers whose ω residue is citric acid and the like are more preferred. Typical example of the "lactic acid-glycolic acid" is polylactic acid and the like.

The weight average molecular weight of the "biodegradable polymer having two or more carboxylic groups at its end" is usually about 200 to about 100,000, preferably about 300 to about 50,000, more preferably about 500 to about 10,000.

The degree of dispersion (weight average molecular weight/number average molecular weight) of the "biodegradable polymer having two or more carboxylic groups at its end" is usually about 1.1 to about 4.0, preferably about 1.2 to 3.5.

Where the ω residue of the "biodegradable polymer having two or more carboxylic groups at its end" is an α,α-dicarboxylic group, the end carboxylic group mass per unit mass of polymer is usually about 30 to about 20,000 µmol/g, preferably about 60 to about 5,000 µmol/g, more preferably about 100 to about 1,000 µmol/g.

The above-mentioned "weight average molecular weight", "number average molecular weight" and "degree of dispersion" mean molecular weights in terms of polystyrene measured by gel permeation chromatography (GPC) using 11 kinds of polystyrenes having weight average molecular weights of 455645, 354000, 98900, 66437, 37200, 17100, 9830, 5870, 2500, 1303 and 504 as reference and a calculated degree of dispersion, respectively. In the measurement, a high performance GPC apparatus (manufactured by Tosoh Corporation, HLC-8120GPC) and a GPC column KF804L×2 (manufactured by SHOWA DENKO K.K.), while using chloroform for the mobile phase.

The "end carboxylic group mass" is determined by quantification of end group based on a labeling method. More specifically, for the case of a polymer whose ω residue is tartronic acid, the biodegradable polymer (W mg) is dissolved in 5N HCl/acetonitrile (v/v=4/96) mixture (2 mL), then 0.01 M o-nitrophenylhydrazine (ONPH) solution (5N HCl/acetonitrile/ethanol=1.02/35/15) (2 mL) and 0.15 M EDC solution (pyridine/ethanol=4v/96v) (2 mL) are added thereto, and the resultant solution is allowed to react at 40°C for 30 minutes, followed by distillation of the solvent. After washing the residue with water (four times), the residue is dissolved in acetonitrile (2 mL), and 0.5 mol/L potassium hydroxide solution in ethanol (1 mL) is added to allow reaction at 60°C for 30 minutes. Diluting the reaction mixture with 1.5 N NaOH to render Y mL, and absorbance A(/cm) at 544 nm is measured using 1.5 N NaOH as a control. On the other hand, using an aqueous solution of tartronic acid as a standard substance, an amount of free carboxylic groups (C mol/L) is determined by NaOH titration. Defining an absorbance at 544 nm for tartronic acid hydrazide which is obtained by ONPH labeling method as B (/cm), an amount of free carboxylic groups [COOH] of the polymer whose ω residue is tartronic acid can be calculated according the following expression:

[COOH] (mol/g)= (AYC)/(WB)

The end carboxylic group mass can also be calculated by dissolving the biodegradable polymer in a toluene-acetone-methanol mixture solvent, and titrating the resultant solution with an alcoholic potassium hydroxide using phenolphthalein as an indicator.

Examples of the "salt" of the "biodegradable polymer having two or more carboxylic groups at its end" include salts with inorganic bases (e.g., alkaline metals such as sodium and potassium, alkaline earth metals such as calcium and magnesium), salts with organic bases (e.g., organic amines such as triethylamine, basic amino acids such as arginine), and salts and complex salts with transition metals (e.g., zinc, iron, copper, etc.).

"Biodegradable polymer having two or more carboxylic groups at its end or a salt thereof" can be produced according to a per se known method or analogous methods thereto. In the case of a biodegradable polymer having two or more carboxylic groups at its ω end, for example, a biodegradable polymer having two or more protected carboxylic groups at its ω end is obtained in accordance with either of the manners (1) and (2) described below, and then the polymer is subjected to deprotection.
(1) In the presence of a hydroxypolycarboxylic acid derivative whose carboxylic groups are protected, a cyclic ester compound is subjected to polymerization using a polymerization catalyst, to obtain a polymer having two or more protected carboxylic groups at its ω end.
   Examples of the "a hydroxypolycarboxylic acid derivative whose carboxylic groups are protected" include hydroxypolycarboxylic acid derivatives whose carboxylic group (-COOH) is amidated (-CONH₂) or esterified (-COOR¹¹). Among these, hydroxypolycarboxylic acid derivatives whose carboxylic group is esterified are preferred.
   Examples of R¹¹ include C₁₋₆ alkyl groups (e.g., methyl, ethyl, propyl, isopropyl, butyl, tert-butyl, etc.), C₃₋₈ cycloalkyl groups (e.g., cyclopentyl, cyclohexyl, etc.), C₆₋₁₂ aryl groups (e.g., phenyl, α-naphthyl, etc.), C₇₋₁₄ aralkyl groups (e.g., phenyl-C₁₋₂ alkyl groups such as benzyl and phenetyl; α-naphtyl-C₁₋₂ alkyl groups such as α-naphthylmethyl) and the like. Among these, a tert-butyl group, a benzyl group and the like are preferred.
   Preferred concrete examples of the "hydroxypolycarboxylic acid derivative whose carboxylic groups are protected" include dibenzyl tartronate, di-tert-butyl 2-hydroxyethylmalonate, tribenzyl citrate and the like.
   Examples of the "polymerization catalyst" include organic tin-based catalysts (e.g., tin octylate, di-n-butyl tin dilaurylate, tetraphenyl tin and the like), aluminum-based catalysts (e.g., triethylaluminum, etc.), zinc-based catalysts (e.g., diethylzinc, etc.) and the like. Among these aluminum-based catalysts and zinc-based catalysts are preferred, with zinc-based catalysts being more preferred. As the solvent for the polymerization catalyst, benzene, hexane, toluene and the like are used, and among these hexane, toluene and the like are preferred.
   The "cyclic ester compound" means cyclic compounds having at least one ester bond in the molecule. Concretely, cyclic monoester compounds (lactones) and cyclic diester compounds (lactides) and the like are exemplified. Examples of the "cyclic monoester compound" include 4-membered cyclic lactones (e.g., β-propiolactone, β-butyrolactone, β-isovalerolactone, β-caprolactone, β-isocaprolactone, β-methyl-β-valerolactone, etc.), 5-membered cyclic lactones (e.g., γ-butyrolactone, γ-valerolactone, etc.), 6-membered cyclic lactones (e.g.; δ-valerolactone), 7-membered cyclic lactones (e.g., ε-caprolactone), p-dioxanone, 1,5-oxepane-2-on and the like. Examples of the "cyclic diester compound" include compounds represented by the formula: 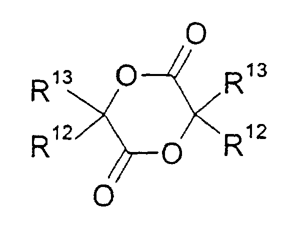 [wherein, R¹² and R¹³ are the same or different and represent, independently, a hydrogen atom or a C₁₋₆ alkyl group (e.g., methyl, ethyl, propyl, isopropyl, butyl, tert-butyl, etc.)] and the like. Among them, compounds wherein R¹² is a hydrogen atom and R¹³ is a methyl group, or compounds wherein R¹² and R¹³ are hydrogen atoms are preferred. Concrete examples include glycoside, L-lactide, D-lactide, DL-lactide, meso-lactide, 3-methyl-1,4-dioxane-2,5-dione (including optically active configurations), etc.
   Polymerization can be achieved by the bulk polymerization method, in which the reaction is carried out with the reaction mixture in a melted state, and the solution polymerization method, in which the reaction is carried out with the reaction mixture dissolved in an appropriate solvent. As the solvent, for example, benzene, toluene, xylene, decalin, dimethylformamide and the like are used, and among these, toluene, xylene and the like are preferred.
   Although polymerization temperature is not particularly limited, it exceeds the temperature at which the reaction mixture is melted at the time of starting the reaction, and is normally in the range of 100 to 300°C, for bulk polymerization, and is normally in the range from room temperature to 150°C for solution polymerization. If the reaction temperature exceeds the boiling point of the reaction mixture, refluxing with a condenser can be used, or the reaction can be carried out in a pressure-resistant container.
   Polymerization time is determined as appropriate, in consideration of polymerization temperature and other reaction conditions, the physical properties of the desired polymer, etc., from 10 minutes to 72 hours, for example.
   After completion of the reaction, the reaction mixture may be dissolved in an appropriate solvent (e.g., acetone, dichloromethane, chloroform, etc.) if necessary, and after polymerization is stopped with an acid (e.g., hydrochloric acid, acetic anhydride, trifluoroacetic acid, etc.), the desired product may be precipitated by, for example, mixing the solution in a solvent that does not dissolve the desired product (e.g., alcohols, water, ether, isopropylether, etc.) according to routine techniques, whereby a polymer having protected carboxylic groups at its ω end is isolated.
(2) The hydroxypolycarboxylic acid derivative whose carboxylic groups are protected and a biodegradable polymer obtained by a per se known method (e.g., catalyst-free condensation polymerization via dehydration) are subjected to condensation reaction using a dehydrating agent and/or an activator of functional group if necessary, to give a polymer having two or more protected carboxylic groups at its ω end.

The carboxylic group involved in the "condensation reaction" may be activated by a known manner, or activation of carboxylic group may be conducted at the time of condensation reaction. Examples of the activating manner include forming an active ester (e.g., ester with a substituted phenols (e.g., pentachlorophenol, 2,4,5-trichlorophenol, 2,4-dinitrophenol, p-nitrophenol, etc.) or N-substituted imides (e.g., N-hydroxy-5-norbornene-2,3-dicarboximide, N-hydroxysuccinimide, N-hydroxy-1,2,3-benzotriazol, etc.)), carboxylic anhydride or azide with carboxylic acid of the starting material, acyl chloride method, oxidation-reduction method (Mukaiyama method), mixed acid anhydride method, N,N'-dicyclohexylcarbodiimide method, N,N'-dicyclohexylcarbodiimide-additives method, a method using Woodward reagent K, a method using benzotriazol-1-yl-oxytris(dimethylamino)-phosphonium hexafluorophosphate (BOP reagent) and the like.

The condensation reaction is generally carried out in the solvent which does not prevent the reaction. Examples of such solvent include amides (e.g., dimethyl formamide, etc.), ethers (e.g., tetrahydrofuran, dioxane, etc.), halogenated hydrocarbons (e.g., dichloromethane, chloroform, etc.), sulfoxides (e.g., dimethylsulfoxide, etc.), esters (e.g., ethyl acetate), N-methylpyrrolidone, N-methylmorpholine, and the like. The reaction temperature is preferably in a range of about -30°C to about 50°C. The reaction temperature is more preferably about 0°C to about 40°C. The reaction time is about 10 minutes to about 24 hours, for example.

As the method for subjecting the "biodegradable polymer having two or more protected carboxylic groups at its ω end" obtained in the above (1) or (2) to "deprotection", a per se know method can be recited. Any method is acceptable insofar as it can remove the protected groups without influencing on ester bonds of poly(hydroxycarboxylic acid), and examples of which include reduction, acid degradation and the like.

Reduction includes catalytic reduction using a catalyst (e.g., palladium-carbon, palladium-black, platinum oxide, etc.), reduction with sodium in liquid ammonium, reduction with dithiothreitol and the like. For example, in the case of subjecting a polymer having carboxylic groups protected with benzyl groups at its ω end to catalytic reduction, concretely after dissolving the polymer in ethyl acetate, dichloromethane, chloroform and the like, palladium carbon is added thereto followed by hydrogen ventilation at room temperature for about 20 minutes to about 4 hours under vigorous stirring.

Examples of acid degradation include acid degradations with inorganic acids (e.g., hydrogen fluoride, hydrogen bromide, hydrogen chloride, etc.) or organic acids (e.g., trifluoroacetic acid, methanesulfonic acid, trifluoromethanesulfonic acid, etc.) or mixture thereof. If necessary, suitable cation scavenger (e.g., anisole, phenol, thioanisole, etc.) may be added to the reaction mixture on the acid degradation. For example, when a polymer having carboxylic groups protected with tert-butyl groups at its ω end is subjected to acid degradation, concretely, after dissolving the polymer in dichloromethane, xylene, toluene or the like, an appropriate amount of trifluoroacetic acid is added to the solution, or alternatively the polymer is dissolved in trifluoroacetic acid, followed by stirring at room temperature for 1 hour.

The acid degradation reaction is preferably carried out directly after the polymerization reaction of (1) as described above. In such a case, it can also achieve polymerization stopping reaction.

Moreover, if necessary, the biodegradable polymer having carboxylic groups at its ω end obtained by the above deprotection reaction is subjected to an acidic hydrolysis reaction for adjusting the weight average molecular weight, number average molecular weight, or end carboxylic group mass in accordance with the object. Concretely, this can be achieved in accordance with a method described, for example, in EP-A-0839525 or a method in accordance with the method.

The content of the "physiologically active non-peptide substance" in the "composition", especially "sustained-release composition" of the present invention is for example, about 0.1 to about 90% (w/w), preferably about 0.5 to about 80% (w/w), more preferably about 1 to 70% (w/w).

The content of "biodegradable polymer having two or more carboxylic groups at its end or a salt thereof" in the "composition", especially "sustained-release composition" of the present invention is for example, about 10 to about 99.9% (w/w), preferably about 20 to about 95.5% (w/w), more preferably about 30 to about 99% (w/w).

The mass ratio of physiologically active non-peptide substance and biodegradable polymer having two or more carboxylic groups at its end or a salt thereof is, for example, about 1/20 to about 100 moles, preferably about 1/10 to about 50 moles, more preferably about 1/5 to about 10 moles of the polymer or a salt thereof with respect to 1 mole of physiologically active non-peptide substance.

"Biodegradable polymer having two or more carboxylic groups at its end" may be mixed with a biodegradable polymer whose end is a monocarboxylic group.

Examples of the "biodegradable polymer whose end is a monocarboxylic group" include poly α-hydroxycarboxylic acids, etc. Examples of "poly α-hydroxycarboxylic acid" include homopolymers of lactic acid, glycolic acid and the like (polylactide or polyglicolide) and copolymers, etc.

The weight ratio of "biodegradable polymer having two or more carboxylic groups at its end" and "biodegradable polymer whose end is a monocarboxylic group" is for example, 100:0 to 30:70, preferably 100:0 to 50:50.

"Composition", especially "sustained-release composition" according to the present invention can be produced by means of in-water drying method, phase separation method, spray drying method and the like methods.

In the following, a production method for the case where the composition is a sustained-release microcapsule (also referred to as microsphere) which constitutes a preferred embodiment will be described.

In the following production method, drug carries (e.g., gelatin, hydroxylnaphthoic acid, salicylic acid, etc.) may be added in per se known manners as necessary.

### (I) In-water Drying Method

A solution of a biodegradable polymer having two or more carboxylic groups at its end or a salt thereof (hereinafter, abbreviated as "biodegradable polymer") in an organic solvent is first prepared. As the organic solvent, those having a boiling point of less than 120°C are preferred, and examples of such organic solvents include halogenated hydrocarbons (e.g., dichloromethane, chloroform, dichloroethane trichloroethane, carbon tetrachloride, etc.), ethers (e.g., ethylether, isopropylether, etc.), fatty acid esters (e.g., ethyl acetate, butyl acetate, etc.), aromatic hydrocarbons (e.g., benzene, toluene, etc.), alcohols (e.g., ethanol, methanol, etc.), acetonitrile and mixtures thereof. Among these, halogenated hydrocarbons are preferred, with dichloromethane being more preferred. As the mixtures, mixture solutions of halogenated hydrocarbons and alcohols are preferred, with a mixture solution of dichloroethane and ethanol being preferred.

Although the biodegradable polymer concentration in the organic solvent solution varies depending on the molecular weight of the biodegradable polymer, the kind of organic solvent etc., it is normally chosen over the range from about 0.5 to about 80% by weight, preferably about 1 to about 70% by weight, more preferably about 2 to about 60% by weight, for example, when dichloromethane is used as an organic solvent.

A physiologically active non-peptide substance (hereinafter, abbreviated as "physiologically active substance") is dissolved or dispersed in the prepared solution in organic solvent. The upper limit of the weight ratio of physiologically active substance to biodegradable polymer is about 1:0.2, preferably about 1:0.5.

A solubilizing agent may be added to the biodegradable polymer solution in organic solvent for increasing the solubility of the physiologically active agent. Examples of such "solubilizing agent" include substances which are acidic and soluble in the solution containing polymer in organic solvent. Preferred examples include acetic acid, hydroxycarboxylic acids having a benzene ring (e.g., salicylic acid, 3-hydroxy-2-naphthoic acid, 1-hydroxy-2-naphthoic acid, pamoic acid, etc.) and the like. Among these, salicylic acid, 3-hydroxy-2-naphthoic acid, 1-hydroxy-2-naphthoic acid and the like are preferred. Salicylic acid, 3-hydroxy-2-naphthoic acid and the like are more preferred.

Next, the obtained solution in organic solvent containing a composition comprising the biodegradable polymer and the physiologically active substance is added to a water phase, to allow formation of O (oil phase)/ W (water phase) emulsion, followed by evaporation of the solvent in the oil phase, thereby preparing microcapsules. The volume of water phase in this case is generally about 1 to about 10,000 times, preferably about 5 times to about 5,000 times, more preferably about 10 times to about 2,000 times the volume of the oil phase.

In addition to the above, an emulsifier may be added to the water phase. Any substance can be used as such "emulsifier" as far as being able to form a stable O/W emulsion. Concrete examples include anionic surfactants (e.g., sodium oleate, sodium stearate, sodium laurate, etc.), nonionic surfactants (e.g., polyoxyethylsorbitan fatty acid esters (Tween 80, Tween 60, manufactured by Atlas Powder Corporation), polyoxyethylene castor oil derivatives (HCO-60, HCO-50, Nikko Chemicals Co., Ltd), polyvinylpyrrolidone, polyvinyl alcohol, carboxymethyl cellulose, lecithin, gelatin and hyaluronic acid. These may be used alone or in combination of two or more kinds.

The concentration of such "emulsifier" is for example, about 0.01 to 10% by weight, preferably 0.05 to about 5% by weight.

In addition to the above, the water phase may be combined with an osmotic pressure adjustor. As the osmotic pressure adjustor, any material can be used so long as it produces osmotic pressure in an aqueous solution thereof. Examples of the osmotic pressure adjustor include polyhydric alcohols, monohydric alcohols, monosaccharides, disaccharides, oligosaccharide, amino acids or their derivatives and the like. Examples of the above "polyhydric alcohols" include trihydric alcohols (e.g., glycerin, etc.), pentahydric alcohols (e.g., arabitol, xylitol, adonitol, etc.), hexahydric alcohols (e.g., mannitol, sorbitol, dulcitol, etc.) and the like. Among them, hexahydric alcohols are preferred. In particular, mannitol is preferred. Examples of the "monohydric alcohols" include methanol, ethanol, isopropyl alcohol and the like. Among them, ethanol is preferred. Examples of the above "monosaccharides" include pentoses (e.g., arabinose, xylose, ribose, 2-deoxyribose, etc.) and hexoses (e.g., glucose, fructose, galactose, mannose, sorbose, rhamnose, fucose, etc.). Among them, hexoses are preferred. Examples of the above "oligosaccharides" include trisaccharides (e.g., maltotriose, raffinose, etc.) and tetrasaccharides (e.g., stachyose, etc.). Among them trisaccharides are preferred. Examples of the "derivatives" of the above monosaccharides, disaccharides and oligosaccharides include glucosamine, galactosamine, glucuronic acid, galacturonic acid and the like. Examples of the above "amino acids" include any L-amino acids, for example, glycine, leucine and arginine. Among them L-arginine is preferred. These osmotic pressure adjustors may be used alone or in combination of two or more kinds.

The "osmotic pressure adjustor" is used in such a concentration that the osmotic pressure of the external water phase is about 1/50 to about 5 times, preferably about 1/25 to about 3 times the osmotic pressure of saline.

The removal of the organic solvent can be carried out by conventional methods. For example, it is carried out by evaporating the organic solvent by stirring with a propeller-type stirrer, magnetic stirrer, etc. under atmospheric pressure or gradually reducing pressure or while controlling degree of vacuum by using a rotary evaporator, etc.

Microcapsules thus obtained are collected by centrifugation or filtration. Then free physiologically active substance, emulsifier and the like attached onto the surface of the microcapsules is washed with distilled water repeatedly several times water, dispersed again in distilled water or the like and subjected to freeze-drying.

During the above production process, an aggregation inhibitor may be added. Examples of the aggregation inhibitor include water-soluble polysaccharides such as mannitol, lactose, glucose, starches (e.g. corn starch), amino acids (e.g., glycine), proteins (e.g., fibrin, collagen) and the like. Among them, mannitol is preferable.

After completion of the freeze-drying, if necessary, water and the organic solvent in the microcapsules may further be removed by heating. Preferably, the heating is conducted at a temperature slightly higher than the intermediate glass transition point of the biodegradable polymer determined using a differential scanning calorimeter when the temperature is increased at a rate of 10 to 20°C per minute. More preferably, the heating is conducted at a temperature in the range of about 30°C higher than the intermediate glass transition temperature of the biodegradable polymer. For example, in the case where lactic acid/glycolic acid polymer is used as the biodegradable polymer, the heating is conducted at a temperature ranging from the intermediate glass transition temperature thereof to the temperature which is higher by 10°C than the glass transition temperature, preferably at a temperature ranging from the intermediate glass transition temperature thereof to the temperature which is higher by 5°C than the glass transition temperature.

Although the heating time differs depending on the amount of the microcapsules and the like, it is generally about 12 hours to about 168 hours, preferably 24 hours to about 120 hours, more preferably 48 hours to about 96 hours after the microcapsules themselves have reached a predetermined temperature.

The heating method is not critical but any procedure conducive to a uniform heating of microcapsules can be employed. As specific examples of such procedure, there may be mentioned heating in a constant-temperature bath, a fluidized bed, a moving bed or a kiln, and microwave heating. The most preferred, of them, is heating in a constant-temperature bath.

### (II) Phase Separation method

To the solution in organic solvent containing the composition comprising the physiologically active substance and the biodegradable polymer as described in the above (I), a coacervating agent is gradually added under stirring to precipitate and solidify the microcapsules. Any coacervating agent can be used, as long as it is a polymeric, mineral oil or vegetable oil compound miscible with the solvent for the high molecular polymer and that does not dissolve the polymer for capsulation. Concrete examples of such coacervating agents include silicon oil, sesame oil, soybean oil, corn oil, cotton seed oil, coconut oil, linseed oil, mineral oil, n-hexane and n-heptane. These may be used in combination of two or more kinds.

The use amount of the coacervating agent is about 0.01 to 1,000 times, preferably about 0.05 to 500 times, more preferably about 0.1 to 200 times the volume of the oil phase.

After collecting the microcapsules thus obtained, the microcapsules are (i) washed with heptane or the like repeatedly to remove components other than the composition comprising the physiologically active substance and the biodegradable polymer (such as coaservating agent), followed by drying under reduced pressure; or alternatively (ii) washed with distilled water repeatedly and dispersed again in distilled water and the like, followed by freeze-drying and heat-drying.

### (III) Spray-drying method

For preparing microcapsules, the solution or dispersion in organic solvent containing the composition comprising the physiologically active substance and the biodegradable polymer as described in the above (I), is sprayed via a nozzle into the drying chamber of a spray drier to volatilize the organic solvent in fine droplets in a very short time. Examples of the above-mentioned nozzle are a binary-fluid nozzle, a pressure nozzle and a rotary disk nozzle. Thereafter, if necessary, freeze-drying and heat-drying may be conducted after conducting washing in the same manner as described in the above water drying method (I).

Examples of production method in the case where the composition, in particular, the sustained-release composition is in the form of, for example, microparticles will be described below.

After evaporating organic solvent and water in the solution or dispersion in organic solvent containing the composition comprising the physiologically active substance and the biodegradable polymer described in the above (I) while controlling the degree of vacuum using a rotary evaporator or the like for drying and solidifying, the resultant product is grained by a jet mill or the like to obtain microparticles. Further the grained microparticles may be washed in the same manner as described in the above (I), followed by freeze-drying and heat-drying.

For use as suspended-injections, for example, the composition, in particular, the sustained-release composition of the present invention may have any particle size insofar as the degree of dispersion and puncturability are satisfied. For example, the average particle size is about 0.1 to 300 µm, preferably about 0.5 to 150 µm, and more preferably about 1 to 100 µm. The average particle size can be determined, for example, using a Laser-analyzed type particle size distribution measuring machine (SALD 2000A, SHIMADZU) and the like in a per se known manner.

The composition, especially stained-release composition of the present invention may be, directly or as a raw material, formulated into various dosage forms such as intramuscular-, subcutaneous- or organ-injectable or indwellable forms, nasal-, rectal or uterine-transmucosal preparations, oral preparations (e.g., capsules such as hard capsule and soft capsule, solid preparations such as in granules and powder, liquid preparations such as syrup, emulsion and suspension). Among them, injectable preparations are particularly preferred.

For instance, in the case where the composition, in particular, the sustained-release composition of the present invention is an injectable, the composition is made into an aqueous suspension together with dispersing agents [e.g., surfactants such as Tween 80 and HCO-60, polysaccharides such as sodium hyaluronate, carboxymethyl cellulose, sodium alginate], preservatives (e.g. methyl paraben, propyl paraben, etc.), isotonizing agents (e.g. sodium chloride, mannitol, sorbitol, glucose, proline, etc.) and other additives or into an oil dispersion by dispersing together with vegetable oils such as sesame oil and corn oil.

As a method for making the composition, in particular, the sustained-release composition according to the present invention into a sterile preparation, conducting the whole producing process under sterile conditions, sterilizing with gamma ray, adding an antiseptic agent and the like are exemplified without limited thereto.

With low toxicity, the composition, especially sustained-release composition of the present invention can be used in mammals (e.g., human, bovine, pig, dog, cat, mouse, rat, rabbit, etc.) as safe pharmaceutical agents and the like.

The composition, in particular, the sustained-release composition of the present invention may be used as preventive and therapeutic agents in accordance with the kind of the contained physiologically active substance. For example, in the case where the physiologically active substance is a GnRH antagonist, the composition of the present invention is useful for preventing and/or treating sex hormone-dependent cancers (e.g., prostatic cancer, uterine cancer, breast cancer, pituitary tumor, etc.), bone metastasis of the sex hormone-dependent cancers, prostatic hypertrophy, hysteromyoma, endometriosis, precocious puberty, amenorrhea, premenstrual syndrome, multilocular ovary syndrome, pimples, alopecia, Alzheimer's disease (Alzheimer's disease, Alzheimer's senile dementia and mixed type thereof), etc. The composition, in particular, the sustained-release composition of the present invention is also useful for the regulation of reproduction in males and females (e.g., pregnancy regulators, menstruation cycle regulators, etc.). The composition, in particular, the sustained-release composition of the present invention may also be used as a male or female contraceptive, or as a female ovulation inducer. Based on its rebound effect after withdrawal, the composition, in particular, the sustained-release composition of the present invention can be used to treat infertility. Also the composition, in particular, the sustained-release composition of the present invention can be used for preventing and/or treating benign or malignant tumor which is independent of sex hormone and sensitive to LH-RH.

In addition, the composition, in particular, the sustained-release composition of the present invention is useful for regulation of animal estrous, improvement of meat quality and promotion of animal growth in the field of animal husbandry. The composition, in particular, the sustained-release composition of the present invention can be also useful as a fish spawning promoter.

The composition, in particular, the sustained-release composition of the present invention can also be used to suppress the transient rise in plasma testosterone concentration (flare phenomenon) observed in administration of a GnRH super-agonist such as leuprorelin acetate. The composition, in particular, the sustained-release composition of the present invention can be used in combination with a GnRH super-agonist such as leuprorelin acetate, gonadrelin, buserelin, triptorelin, goserelin, nafarelin, histrelin, deslorelin, meterelin, lecirelin, and so on. Among them, preferred is leuprorelin acetate.

It is also beneficial to use the composition, in particular, the sustained-release composition of the present invention in combination with at least one member selected from among the steroidal or nonsteroidal androgen antagonist or antiestrogen, chemotherapeutic agent, GnRH antagonistic peptide, 5α-reductase inhibitor, α-receptor inhibitor, aromatase inhibitor, 17β-hydroxysteroid dehydrogenase inhibitor, adrenal androgen production inhibitor, phosphorylase inhibitor, drug for hormone therapy, and drug antagonizing growth factor or its receptor, among others.

Examples of the "chemotherapeutic agent" mentioned above include ifosfamide, UTF, adriamycin, peplomycin, cisplatin, cyclophosphamide, 5-FU, UFT, methotrexate, mitomycin C, mitoxantrone, taxotere, and the like.

Examples of the "GnRH antagonistic peptide" mentioned above include non-oral GnRH antagonistic peptides such as cetrorelix, ganirelix, abarelix, and the like.

Examples of the "adrenal androgen production inhibitor" mentioned above include lyase (C_{17,20} -lyase) inhibitors, and the like.

Examples of the "phosphorylase inhibitor" mentioned above include tyrosine phosphorylase inhibitor, and the like.

Examples of the "drugs for hormone therapy" include antiestrogens, progesterons (e.g., MPA, etc.), androgens, estrogens and androgen antagonists, and the like.

The "growth factor" may be any substance that promotes proliferation of cells and generally includes peptides with molecular weights less than 20,000 which express the action at low concentrations through binding to receptors. Specifically, there can be mentioned (1) EGF (epidermal growth factor) or substances having the substantially the same activity (e.g., EGF, heregulin (HER2 ligand), etc.), (2) insulin or substances having substantially the same activity (e.g., insulin, IGF (insulin-like growth factor)-1 IGF-2, etc.), (3) FGF (fibroblast growth factor) or substances having substantially the same activity (αFGF, βFGF, KGF (keratinocyte growth factor), HGF (hepatocyte growth factor), FGF-10, etc.), and (4) other growth factors (e.g., CSF (colony stimulating factor), EPO (erythropoietin), IL-2 (interleukin-2), NGF (nerve growth factor), PDGF (platelet-derived growth factor) and TGFβ (transforming growth factor β), etc.), among others.

The "growth factor receptor" mentioned above may be any receptor capable of binding the growth factor, including EGF receptor, heregulin receptor (HER2), insulin receptor-1, insulin receptor-2, IGF receptor, FGF receptor-1, FGF receptor-2, etc.

Examples of the drug antagonizing the growth factor include herceptin (anti-HER2 receptor antibody) and the like.

Examples of the drug antagonizing the growth factor or growth factor receptor include herbimycin, PD153035 (see Science, 265 (5175) p.1093, (1994)) and the like.

As a further class of drugs antagonizing the growth factor or growth factor receptor includes HER2 antagonists. The HER2 antagonist may be any substance that inhibits the activity of HER2 (e.g., phosphorylating activity), thus including an antibody, a low molecular compound (synthetic or natural product), an antisense, a HER2 ligand, heregulin, and any of them as partially modified or mutated in structure. Moreover, it may be a substance which inhibits HER2 activity by antagonizing HER2 receptor (e.g. HER2 receptor antibody). Examples of the low molecular compound having HER2 antagonizing activity include, for example, compounds described in WO 98/03505, namely 1-[3-[4-[2-((E)-2-phenylethenyl)-4-oxazolylmethoxy]phenyl]propyl]-1,2,4-triazole and so on.

For prostatic hypertrophy, examples of such combination include the composition, in particular, the sustained-release composition of the present invention in combination with the GnRH super-agonist, androgen antagonist, antiestrogen, GnRH antagonistic peptide, 5α-reductase inhibitor, α-receptor inhibitor, aromatase inhibitor, 17β-hydroxysteroid dehydrogenase inhibitor, adrenal androgen production inhibitor, phosphorylase inhibitor, and so on.

For prostatic cancer, examples of such combination include the composition, in particular, the sustained-release composition of the present invention in combination with the GnRH super-agonist, androgen antagonist, antiestrogen, chemotherapeutic agent (e.g., ifosfamide, UTF, adriamycin, peplomycin, cisplatin, etc.), GnRH antagonistic peptide, aromatase inhibitor, 17β-hydroxysteroid dehydrogenase inhibitor, adrenal androgen production inhibitor, phosphorylase inhibitor, drug for hormone therapy such as estrogens (e.g., DSB, EMP, etc.), androgen antagonist (e.g., CMA. etc.), drug antagonizing growth factor or its receptor, and so on.

For breast cancer, examples of such combination includes the composition, in particular, the sustained-release composition of the present invention in combination with the GnRH super-agonist, antiestrogen, chemotherapeutic agent (e.g., cyclophosphamide, 5-FU, UFT, methotrexate, adriamycin, mitomycin C, mitoxantrone, etc.), GnRH antagonistic peptide, aromatase inhibitor, adrenal androgen production inhibitor, phosphorylase inhibitor, drug for hormone therapy such as antiestrogen (e.g., tamoxifen, etc.), progesterons (e.g., MPA, etc.), androgens, estrogens, etc., drug antagonizing growth factor or its receptor, and so on.

The above drugs may be administered to the same subject concurrently or at some interval, with the composition, in particular, the sustained-release composition of the present invention. Furthermore, the composition, in particular, the sustained-release composition of the present invention may be administered prior to administration of the GnRH super-agonist such as leuprorelin acetate so as to conduct a treatment while preventing occurrence of flare phenomenon.

The dose of the composition, in particular, the sustained-release composition of the present invention differs in accordance with the kind, content, dosage form of the physiologically active substance and duration of release of the physiologically active substance, objective disease, objective animal and the like, however, it can be an effective amount of the physiologically active substance. A dosage of the physiologically active substance per one administration, is for example, about 0.01 mg to 20 mg/kg of body weight, preferably about 0.05 mg to 5 mg/kg of body weight for an adult person (body weight: 60 kg) when the composition is a monthly formulation.

Dose for one administration of the composition, in particular, the sustained-release composition according to the present invention is about 0.05 mg to 50 mg/kg, preferably about 0.1 mg to 30 mg/kg per an adult person (body weight: 60 kg).

The number of administrations can be appropriately selected according to the kind, content and dose form of the physiologically active substance, duration of release of the physiologically active substance, objective disease, objective animal and the like, for example every several weeks, once a month, every several months (e.g., every 3 months, 4 months, 6 months, etc.).

The release time of the physiologically active substance from the composition, in particular, the sustained-release composition according to the present invention is not particularly limited since it is variable in accordance with the kind of the physiologically active substance, dose form of the composition, dose and site of administration, and is for example, 12 hours to 1 year, preferably 24 hours to 8 months, and more preferably 1 week to 4 months.

The composition, in particular, the sustained-release composition of the present invention is preferably those having a glass transition point (Tg) higher than that of the biodegradable polymer having two or more carboxylic groups at its end by more than about 10°C, preferably by more than about 12°C, and more preferably by more than about 15°C from the view point of the safety. In this context, "glass transition point" is measured by means of a differential scanning calorimeter (DSC7, manufactured by Perkin-Elmer). Concretely, compositions of the present invention having a grass transition point of about 53 to 58°C with high safety can be obtained using biodegradable polymers (for example, end tartronic acid PLA obtained in the Reference example 9 or Reference example 10) having a glass transition point of about 31 to 42°C.

Also the present composition is preferably a sustained-release composition. That is, it is preferred that when the physiologically active non-peptide substance is water-insoluble or slightly water-soluble, release rate of the physiologically active non-peptide substance is accelerated.

In the following, the present invention will be explained in more detail by way of reference examples, working examples and experimental examples, however, these are not intended to limit the present invention.

### EXAMPLES

### Reference example 1

### 2-Amino-4-methyl-5-(4-nitrophenyl)thiophene-3-carboxylic acid ethyl ester

A mixture of 4-nitrophenylacetone (35.0 g, 195 mmol), ethyl cyanoacetate (23.8 g, 195 mmol), ammonium acetate (3.1 g, 40 mmol) and acetic acid (9.1 mL, 159 mmol) was refluxed for 24 hours while removing water using a Dean-Stark apparatus. After cooling, the reaction mixture was concentrated under reduced pressure and the residue was partitioned with dichloromethane and aqueous solution of sodium bicarbonate. After washing the organic phase with brine and drying over MgSO₄, the solvent was distilled off under reduced pressure. The residue was purified on a silica gel chromatography. The resultant oily product was dissolved in ethanol and sulfur (5.0 g, 160 mmol) and diethyl amine (16.0 mL, 160 mmol) were added thereto, and the mixture was stirred at 60 to 70°C for 2 hours. After cooling, the reaction mixture was concentrated under reduced pressure, and the residue was partitioned with dichloromethane and aqueous solution of sodium bicarbonate. After washing the organic phase with brine and drying over MgSO₄, the solvent was distilled off under reduced pressure. The residue was purified on a silica gel chromatography, and crystallized from ether-hexane, to give the title compound as a red sheet crystals (22.2 g, 52%).
mp: 168-170°C (recrystallized from ether-hexane)

| Element Analysis for C₁₄H₁₄N₂O₄S; | | | |
|---|---|---|---|
| | C(%) | H(%) | N(%) |
| Calcd. | 54.89; | 4.61; | 9.14 |
| Found | 54.83; | 4.90; | 9.09 |

¹H-NMR(200 MHz, CDCl₃)δ: 1.39 (3H, t, J = 7.1 Hz), 2.40 (3H, s), 4.34 (2H, q, J = 7.1 Hz), 6.27(2H, br), 7.48 (2H, d, J = 8.7 Hz), 8.23 (2H, d, J = 8.7 Hz).
IR (KBr): 3446, 3324, 1667, 1580, 1545, 1506, 1491, 1475, 1410, 1332 cm⁻¹.

### Reference example 2

### 5-Methyl-6-(4-nitrophenyl)-3-phenylthieno[2,3-d]pyrimidine-2,4(1H,3H)-dione

Phenylisocyanate (2.66 mL, 24.48 mmol) was added to a solution of the compound obtained in Reference example 1 (5.00 g, 16.32 mmol) in pyridine (30 mL). The mixture was stirred at 45°C for 6 hours and concentrated under reduced pressure to give a residue, which was then rendered a solution in ethanol (6 mL). To this solution, 28% sodium methoxide (7.86 g, 40.80 mmol) was added, and the mixture was stirred at the room temperature for 2 hours, followed by addition of 2N hydrochloric acid (25 mL, 50 mmol) and distillation of ethanol solvent under reduced pressure. The resultant residue was filtrated, washed with water-ethanol and recrystallized from ethanol after drying under reduced pressure to give the title compound as yellow powder (6.09 g, 98%).
mp: >300°C

| Element Analysis for C₁₉H₁₃N₃O₄S·0.3H₂O | | | |
|---|---|---|---|
| | C(%) | H(%) | N(%) |
| Calcd. | 59.30; | 3.56; | 10.92 |
| Found | 59.56; | 3.52; | 10.93 |

¹H-NMR(300 MHz, DMSO-d₆) δ: 2.50 (3H, s), 7.31-7.46 (5H, m), 7.78(2H, d, J = 8.8 Hz), 8.32 (2H, d, J = 8.8 Hz), 12.50 (1H, s) .
IR(KBr) : 1715, 1657, 1593, 1510 cm⁻¹.

### Reference example 3

### 1-(2,6-Difluorobenzyl)-5-methyl-6-(4-nitrophenyl)-3-phenylthieno[2,3-d]pyrimidine-2,4(1H,3H)-dione

Potassium carbonate (19.00 g, 0.138 mol), potassium iodide (22.90 g, 0.138 mol) and 2,6-difluorobenzyl chloride (22.40 g, 0.138 mol) were added to a solution of the compound obtained in Reference example 2 (52.54 g, 0.131 mol) in dimethylformamide (1.0 L), and the mixture was stirred at room temperature for 2 hours. A residue obtained after concentration of the reaction mixture was partitioned with chloroform and brine. The water layer was extracted with chloroform, and the combined extract was washed with brine and dried over MgSO₄, followed by distillation of solvent under reduced pressure. The resultant residue was purified on a silica gel chromatography, to give the title compound as pale yellow crystals (61.50 g, 93%).
mp: 280-282°C

| Element Analysis: for C₂₆H₁₇N₃O₄SF₂ | | | |
|---|---|---|---|
| | C(%) | H(%) | N(%) |
| Calcd. | 61.78; | 3.39; | 8.31 |
| Found | 61.67; | 3.46; | 8.21 |

¹H-NMR(300 MHz,CDCl₃) δ: 2.57 (3H, s), 5.38 (2H, s), 6.94 (2H, d, J = 8.1 Hz), 7.42-7.58 (8H, m), 8.29 (2H, d, J = 8.8 Hz).
IR(KBr): 1719, 1669, 1524, 1473 cm⁻¹

### Reference example 4

### 5-Bromomethyl-1-(2,6-difluorobenzyl)-6-(4-nitrophenyl)-3-phenylthieno[2,3-d]pyrimidine-2,4(1H,3H)-dione

A mixture of the compound obtained in Reference example 3 (30.34 g, 0.060 mol), N-bromosuccinimide (12.81 g, 0.072 mol), α,α'-azobisisobutyronitrile (1.15 g, 0.007 mol) and chlorobenzene (450 mL) was stirred at 85°C for 3 hours. After cooling, the reaction mixture was washed with brine and dried over MgSO₄, followed by distillation of the solvent under reduced pressure. The resultant residue was recrystallized from ethyl acetate to give the title compound as yellow needle crystals (80.21 g, 100%).
mp: 228-229°C
¹H-NMR(300 MHz,CDCl₃) δ: 4.77 (2H, s), 5.38 (2H, s), 6.96 (2H, t, J = 8.1 Hz), 7.29-7.58 (6H, m), 7.79 (2H, d, J = 8.5 Hz), 8.35 (2H, d, J = 8.5 Hz).
IR(KBr): 1721, 1680, 1524, 1473, 1348 cm⁻¹.
FAB-Mass m/z 586 (MH)⁺

### Reference example 5

### 5-(N-Benzyl-N-methylaminomethyl)-1-(2,6-difluorobenzyl)-6-(4-nitrophenyl)-3-phenylthieno[2,3-d]pyrimidine-2,4(1H,3H)-dione

Ethyldiisopropylamine (27.00 mL, 0.155 mol) and benzylmethylamine (18.45 mL, 0.143 mol) were added to a solution of the compound obtained in Reference example 4 (80.00 g, 0.119 mol) in dimethylformamide (600 mL) under cooling with ice. After stirring at room temperature for 2 hours, the residue obtained by concentration of the reaction mixture was partitioned with ethyl acetate and saturated aqueous solution of sodium bicarbonate. The water layer was extracted with ethyl acetate, and the combined organic layer was dried over MgSO₄, followed by distillation of the solvent under reduced pressure. The resultant residue was purified on a silica gel chromatography to give a yellow oily substance (74.90 g, 100%), which was recrystallized from ethyl acetate to give the title compound as yellow needle crystals.
mp: 173-174°C

| Element Analysis for C₃₄H₂₆N₄O₄SF₂·0.5H₂O | | | |
|---|---|---|---|
| | C(%) | H(%) | N(%) |
| Calcd. | 64.45; | 4.29; | 8.84 |
| Found | 64.50; | 4.24; | 8.82 |

¹H-NMR(300 MHz,CDCl₃) [free amine] δ: 1.31 (3H, s), 3.60 (2H, s), 3.96 (2H, s), 5.39 (2H, s), 6.95 (2H, t, J = 8.2 Hz), 7.18-7.55 (11H, m), 8.02 (2H, d, J = 9.0 Hz), 8.26 (2H, d, J = 9.0 Hz).
IR(KBr][hydrochloride]: 1719, 1678, 1597, 1520 cm⁻¹.

### Reference example 6

### 6-(4-Aminophenyl)-5-(N-benzyl-N-methylaminomethyl)-1-(2,6-difluorobenzyl)-3-phenylthieno[2,3-d]pyrimidine-2,4(1H,3H)-dione

1 M Hydrogen chloride-ether (14.4 mL, 14.4 mmol) and 10% palladium carbon powder (300 mg) was added to a solution of the compound obtained in Reference example 5 (3.00 g, 4.80 mmol) in formic acid (30 mL) under cooling with ice, and the mixture was stirred at room temperature for 2 hours under normal pressures for allowing hydrogenation. The reaction mixture was filtered through Celite, and the residue obtained by concentration of filtrate under reduced pressure was partitioned with dichloromethane and saturated aqueous solution of sodium bicarbonate. The water layer was extracted with dichloromethane, and the combined organic layer was dried over MgSO₄, followed by distillation of the solvent under reduced pressure. The resultant residue was purified on a silica gel chromatography to give the title compound as white crystals (2.41 g, 84%).
mp: 205-207°C

| Element Analysis: C₃₄H₂₈N₄O₂SF₂·0.1AcOEt·1.2H₂O | | | |
|---|---|---|---|
| | C(%) | H(%) | N(%) |
| Calcd. | 66.09; | 5.03; | 8.96 |
| Found | 66.93; | 4.94; | 8.67 |

¹H-NMR(300 MHz, CDCl₃) δ: 2.05(3H, s), 3.56 (2H, s), 3.83 (2H, br), 3.88 (2H, s), 5.36 (2H, s), 6.70 (2H, d, J = 8.8 Hz), 6.88-6.94 (2H, m), 7.21-7.31 (8H, m), 7.41-7.53 (5H, m).
IR(KBr): 1715, 1657, 1628, 1537 cm⁻¹.

### Reference example 7

### 5-(N-Benzyl-N-methylaminomethyl)-1-(2,6-difluorobenzyl)-6-[4-(3-methoxyureido)phenyl]-3-phenylthieno[2,3-d]pyrimidine-2,4(1H,3H)-dione

Triethylamine (2.34 mL, 16.82 mmol) was added to a solution of the compound obtained in Reference example 6 (5.0 g, 8.41 mmol) in dichloromethane (120 mL) under ice-cooling and the mixture was stirred. To this mixture, N,N'-carbonyldiimidazole (2.73 g, 16.82 mmol) was added under ice-cooling, and the mixture was stirred for 42 hours after allowing the reaction temperature to the room temperature. Again under ice-cooling, O-methylhydroxylamine hydrochloride (7.02 g, 84.08 mmol) and triethylamine (11.7 mL, 84.08 mmol) were added to the mixture. After allowing the reaction temperature to the room temperature, the reaction mixture was stirred for 3 hours. The reaction mixture was partitioned with chloroform and saturated aqueous solution of sodium bicarbonate. The water layer was extracted with chloroform and the combined extract was washed with brine, followed by drying over MgSO₄ and distillation of the solvent under reduced pressure. The resultant residue was purified on a silica gel chromatography to give a pale yellow solid which was then recrystallized from chloroform-ether to give the title compound as white crystals (4.52 g, 80%).
mp: 204-205°C

| Element Analysis for C₃₆H₃₁N₅O₄SF₂ | | | |
|---|---|---|---|
| | C(%) | H(%) | N(%) |
| Calcd. | 64.75; | 4.68; | 10.49 |
| Found | 64.61; | 4.67; | 10.31 |

¹H-NMR(300 MHz,CDCl₃) δ: 2.05 (3H, s), 3.57 (2H, s), 3.82 (3H, s), 3.90 (2H, s), 5.37 (2H, s), 6.92 (2H, d, J = 8.2 Hz), 7.16-7.31 (9H, m), 7.42-7.57 (5H, m), 7.63 (1H, s), 7.73 (2H, d, J = 8.8 Hz).
IR(KBr): 3338, 3064, 1717, 1669, 1628, 1591, 1531, 1470 cm⁻¹.

### Reference example 8

### 3-(N-Benzyl-N-methylaminomethyl)-4,7-dihydro-5-isobutyryl-7-(2,6-difluorobenzyl)-2-[4-[(1-hydroxycyclopropyl)-carbonylamino]phenyl]-4-oxothieno[2,3-b]pyridine

Diisopropylethylamine (0.52 g, 4 mmol) and 2-hydroxycyclopropanecarboxylic acid (0.204 g, 2 mmol) was added to a solution of 2-(4-aminophenyl)-7-(2,6-difluorobenzyl)-4,7-dihydro-5-isobutyryl-3-(N-benzyl-N-methylaminomethyl)-4-oxothieno[2,3-b]pyridine (0.57 g, 1.0 mmol) in dichloromethane (10 mL) and the mixture was stirred under ice-cooling. To this mixture was added benzotriazole-1-yloxytris(dimethylamino)-phosphonium hexafluorophosphate (BOP reagent) (1.76 g, 4 mmol). The solution was stirred for 1 hour under ice-cooling, followed by stirring at room temperature for 4 days. After evaporating the reaction mixture under reduced pressure, the resultant residue was partitioned with water (50 mL) and chloroform (50 mL). The water layer was extracted again with chloroform (10 mL). The combined extract was washed with brine, and after drying over MgSO₄, the solvent was distilled off under reduced pressure. The resultant residue was purified on silica gel chromatography, and recrystallized from ether to give yellow powder crystals (0.27 g, 41%).
¹H-NMR(300 MHz, CDCl₃) δ: 1.16-1.20 (2H, m), 1.18 (6H, d), 1.48-1.51 (2H, m), 2.09 (3H, s), 3.64 (2H, s), 3.95 (1H, br s), 4.14 (2H, s), 4.12-4.19 (1H, m), 5.20 (2H, s), 6.99 (2H, t), 7.10-7.25 (5H, m), 7.34-7.46 (1H, m), 7.57 (2H, d), 7.70 (2H, d), 8.21 (1H, s), 8.82 (1H, s).

### Reference example 9

To dibenzyl tartronate cooled to -78°C (2.40 g) was added 1.1 M diethyl zinc toluene solution (3.6 mL) under nitrogen atmosphere, and the resultant solution was allowed to react at room temperature for 20 minutes. To this reaction mixture, DL-lactide (7.04 g) was added and mixed under nitrogen atmosphere and allowed to polymerize at 130°C for 2 hours.

Next, for quenching the polymerization and for deprotection, the reactant was dissolved in trifluoroacetic acid (10 mL), thioanisole (5.64 mL) was added thereto, and the mixture was stirred at -5°C for 1 hour. Furthermore methane sulfonic acid (20 mL) was added to the mixture and the mixture was stirred for another 40 minutes. Then the reaction mixture was mixed into cold isopropyl ether (1.5 L) for collecting polymer by precipitation, followed by purification by reprecipitation with dichloromethane/cold isopropyl ether twice. The purified precipitate was dissolved in dichloromethane and washing was repeated until neutrality was achieved. Next, the dichloromethane solution was concentrated, vacuum dried (40°C, 2 days), to give poly (DL-lactic acid) whose ω residue is tartronic acid. ¹H-NMR analysis revealed that a signal of phenyl hydrogen in a benzyl group completely disappeared, from which completion of deprotection was confirmed. Also as a result of atomic absorption spectrometry, the remaining zinc was below the detection limit (10 ppm), which revealed that the polymerization catalyst was efficiently removed in this manner. As a result of GPC measurement, the weight average molecular weight was 3600, and the degree of dispersion was 1.41. Furthermore, when the polymer was subjected to end-group labeling quantification, strong purple coloring was observed, from which regeneration of carboxylic group by deprotection was confirmed. Also using tartronic acid as a standard substance, the amount of tartronic acid which is an ω residue of the polymer was calculated in terms of dicarboxylic group amount from comparison with the absorbance for tartronic acid hydrazide which is obtained by ONPH labeling method. The calculation result was 378.9 µmol/g.

### Reference example 10

To dibenzyl tartronate cooled to -78°C (2.00 g) was added 1.1 M diethyl zinc toluene solution (3.0 mL) under nitrogen atmosphere, and the resultant solution was allowed to react at room temperature for 20 minutes. To this solution, DL-lactide (19.20 g) was added and mixed under nitrogen atmosphere and allowed to polymerize at 130°C for 2 hours.

Next, for quenching the polymerization and for deprotection, the reactant was dissolved in trifluoroacetic acid (20 mL), thioanisole (4.70 mL) was added to the mixture, and the mixture was stirred for 1 hour at -5°C. Furthermore methane sulfonic acid (20 mL) was added to the mixture and the mixture was stirred for another 20 minutes. Then the reaction mixture was mixed into cold isopropyl ether (1.5 L) for collecting polymer by precipitation, followed by purification by reprecipitation with dichloromethane/cold isopropyl ether twice. The purified precipitate was dissolved in dichloromethane and washing was repeated until neutrality was achieved. Next, the dichloromethane solution was concentrated, vacuum dried (40°C, 2 days), to give poly (DL-lactic acid) whose ω residue is tartronic acid. ¹H-NMR analysis revealed that a signal of phenyl hydrogen in a benzyl group completely disappeared, from which completion of deprotection was confirmed. Also as a result of atomic absorption spectrometry, the remaining zinc was below the detection limit (10 ppm), which revealed that the polymerization catalyst was efficiently removed in this manner. As a result of GPC measurement, the weight average molecular weight was 9600, and the degree of dispersion was 1.68. Furthermore, when the polymer was subjected to end-group labeling quantification, strong purple coloring was observed, from which regeneration of carboxylic group by deprotection was confirmed. Also using tartronic acid as a standard substance, the amount of tartronic acid which is an ω residue of the polymer was calculated in terms of dicarboxylic group amount from comparison with the absorbance for tartronic acid hydrazide which is obtained by ONPH labeling method. The calculation result was 155.3 µmol/g.

### Working example 1

The end tartronic acid PLA obtained in Reference example 9 (weight average molecular weight = 3600, number average molecular weight = 2600) (2.5 g) was added to a mixture solution of dichloromethane (14 mL) and methanol (2 mL) and dissolved. To this solution the compound obtained in Reference example 7 (1.4 g) was added, mixed and dissolved by means of a voltex mixer to give a solution in organic solvent. This solution in organic solvent was poured into 0.1% (w/v) polyvinylalcohol (PVA) (800 mL) whose temperature had been adjusted at 18°C in advance, to render an O/W type emulsion using a turbin-type homomixer. This O/W type emulsion was stirred at room temperature, and dichloromethane was caused to volatile, thereby preparing microcapsules. The obtained microcapsules were collected by a centrifugal separating operation (about 2000 rpm). Next, the microcapsules were washed once with distilled water (400 mL), mannitol (450 mg) was added thereto, followed by freeze-drying, to give powder microcapsules (3.7 g).

### Working example 2

The end tartronic acid PLA obtained in Reference example 10 (weight average molecular weight = 9600, number average molecular weight = 5700) (2.0 g) was added to a mixture solution of dichloromethane (5 mL) and methanol (0.5 mL) and dissolved. To this solution the compound obtained in Reference example 8 (1.0 g) was added, mixed and dissolved by means of a voltex mixer, to give a solution in organic solvent. This solution in organic solvent was poured into 0.1% (w/v) polyvinylalcohol (PVA) (800 mL) whose temperature had been adjusted at 18°C in advance, to render an O/W type emulsion using a turbin-type homomixer. This O/W type emulsion was stirred at room temperature, and dichloromethane was caused to volatile, thereby preparing microcapsules. The obtained microcapsules were collected by a centrifugal separating operation (about 3000 rpm). Next, the microcapsules were washed once with distilled water (400 mL), mannitol (500 mg) was added thereto, followed by freeze-drying, to give powder microcapsules (3.0 g).

### Experimental example 1

(1) Microcapsules A
The lactic acid-glycolic acid polymer having end monocarboxylic acids (lactic acid/glycolic aid = 50/50(mol%), weight average molecular weight = 14000) (6.0 g) was added to a mixture solution of dichloromethane (20 mL) and methanol (2 mL) and dissolved. To this solution the compound obtained in Reference example 7 (1.9 g) was added, mixed and dissolved by means of a voltex mixer, to give a solution in organic solvent. This solution in organic solvent was poured into 0.1% (w/v) polyvinylalcohol (PVA) (800 mL) whose temperature had been adjusted at 18°C in advance, to render an O/W type emulsion using a turbin-type homomixer. This O/W type emulsion was stirred at room temperature, and dichloromethane was caused to volatile, thereby preparing microcapsules. The obtained microcapsules were collected by a centrifugal separating operation (about 2000 rpm). Next, the microcapsules were washed once with distilled water (400 mL), mannitol (450 mg) was added thereto, followed by freeze-drying, to give powder microcapsules A (4.8 g).
(2) Microcapsules B
The lactic acid-glycolic acid polymer having end monocarboxylic acids (lactic acid/glycolic aid = 75/25 (mol%), weight average molecular weight = 14000) (6.5 g) was added to a mixture solution of dichloromethane (20 mL) and methanol (2 mL) and dissolved. To this solution the compound obtained in Reference example 7 (1.8 g) was added, mixed and dissolved by means of a voltex mixer, to give a solution in organic solvent. This solution in organic solvent was poured into 0.1% (w/v) polyvinylalcohol (PVA) (800 mL) whose temperature had been adjusted at 18°C in advance, to render an O/W type emulsion using a turbin-type homomixer. This O/W type emulsion was stirred at room temperature, and dichloromethane was caused to volatile, thereby preparing microcapsules. The obtained microcapsules were collected by a centrifugal separating operation (about 2000 rpm). Next, the microcapsules were washed once with distilled water (400 mL), mannitol (450 mg) was added thereto, followed by freeze-drying, to give powder microcapsules B (7.1 g).
(3) Microcapsules obtained in Working example 1 (Microcapsules 1), the above microcapsules A and microcapsules B were weighed in amounts corresponding to 10 mg of the compound obtained in Reference example 7, and dispersed in 0.5 mL of a dispersing solvent (distilled water in which 0.2% carboxymethyl cellulose, 0.1% polysolvate 80 and 5% mannitol were dissolved), and administered to male SD rats (aged: 6 weeks) subcutaneously in the back by using a needle of 22G with a syringe. After a predetermined time had elapsed from the administration, the rats were scarified and microcapsules remaining in the administration site were collected. The amount of the compound obtained in Reference example 7 contained in the collected microcapsules was measured and the remaining rate obtained by dividing the measured amount by the initial content is shown in Table 1.

**[Table 1]**

| | Microcapsules 1 | Microcapsules A | Microcapsules B |
|---|---|---|---|
| 1 day | 101.2% | 100.7% | 96.9% |
| 1 week | 91.9% | 86.9% | 89.9% |
| 2 weeks | 71.9% | 85.8% | 83.3% |
| 3 weeks | 54.8% | 80.7% | 77.1% |
| 4 weeks | 52.0% | 79.7% | 70.7% |

The results of Table 1 revealed that release of the compound obtained in Reference example 7 which is water-insoluble or slightly water-soluble is significantly slow from the microcapsules A and B produced with the use of lactic acid/glycolic acid polymer having end monocarboxylic aids, and this release does not depend on the ratio of lactic acid/glycolic acid which usually influences on the release speed, so that it is difficult to control the release for such a polymer.

On the other hand, according to the microcapsules 1 of the present invention, it is apparent that release of the compound obtained in Reference example 7 which is water-insoluble or slightly water-soluble is accelerated.

### Experimental example 2

Glass transition points (Tg) of the microcapsules 1, microcapsules A and microcapsules B measured by means of a differential scanning calorimeter (DSC7, manufactured by Perkin-Elmer) were 58.0°C for microcapsules 1, 43.7°C for microcapsules A and 45.4°C for microcapsules B.

The microcapsules 1 whose weight average molecular weight is 3600 according to the present invention exhibited a high Tg which is unexpected for the case where an end is a monocarboxylic group. Since higher Tg is advantageous for stability of formulations, it can be found that the composition of the present invention is superior in stability.

### Experimental example 3

(1) Microcapsules C
The lactic acid-glycolic acid polymer having end monocarboxylic acids (lactic acid/glycolic aid = 75/25 (mol%), weight average molecular weight = 12000) (2.0 g) was added to a mixture solution of dichloromethane (5 mL) and methanol (0.5 mL) and dissolved. To this solution the compound obtained in Reference example 8 (1.0 g) was added, mixed and dissolved by means of a voltex mixer, to give a solution in organic solvent. This solution in organic solvent was poured into 0.1% (w/v) polyvinylalcohol (PVA) (800 mL) whose temperature had been adjusted at 18°C in advance, to render an O/W type emulsion using a turbin-type homomixer. This O/W type emulsion was stirred at room temperature, and dichloromethane was caused to volatile, thereby preparing microcapsules. The obtained microcapsules were collected by a centrifugal separating operation (about 2000 rpm). Next, the microcapsules were washed once with distilled water (400 mL), mannitol (500 mg) was added thereto, followed by freeze-drying, to give powder microcapsules C (3.0 g).
(2) Microcapsules obtained in Working example 2 (Microcapsules 2) and the above microcapsules C were weighed in amounts corresponding to 10 mg of the compound obtained in Reference example 8, and dispersed in 0.5 mL of a dispersing solvent (distilled water in which 0.2% carboxymethyl cellulose, 0.1% polysolvate 80 and 5% mannitol were dissolved), and administered to male SD rats (aged: 6 weeks) subcutaneously in the back by using a needle of 22G with a syringe. After a predetermined time had elapsed from the administration, three animals from each group were scarified and subcutaneous stimulation of the microcapsules was visually observed and recorded. The number of rats which exhibited subcutaneous stimulation after administration of microcapsules among three animals is shown in Table 2.

**[Table 2]**

| | Microcapsules 2 | Microcapsules C |
|---|---|---|
| 1 week | 0 | 2 |
| 2 weeks | 0 | 3 |
| 3 weeks | 0 | 3 |
| 4 weeks | 0 | 3 |
| 5 weeks | 0 | 3 |

The results of Table 2 shows that it is difficult for the microcapsules C produced by using a lactic acid/glycolic acid polymer having end monocarboxylic groups to suppress the subcutaneous stimulation of the compound obtained in Reference example 8.

On the other hand, it is apparent that the microcapsules 2 according to the present invention completely suppress the subcutaneous stimulation of the compound obtained in Reference example 8.

### INDUSTRIAL APPLICABILITY

The composition of the present invention can express secure pharmaceutical effects by enabling increase of the content of a physiologically active non-peptide substance, while controlling or accelerating release of the same. Also when the physiologically active non-peptide substance has a subcutaneous stimulation coming from basic substance, the composition of the present invention is expected to have an activity to cancel the stimulation by means of the strongly acidic group at its end. Also the composition of the present invention has a high grass transition point and hence has great stability.

## Claims

1. A composition comprising a physiologically active non-peptide substance and a biodegradable polymer having two or more carboxylic groups at its end or a salt thereof.

2. A composition in which a physiologically active non-peptide substance and a biodegradable polymer having two or more carboxylic groups at its end or a salt thereof are blended.

3. The composition according to claim 2, wherein the glass transition point (Tg) of the composition is about 10°C or more higher than that of the biodegradable polymer having two or more carboxylic groups at its end.

4. The composition according to claim 1 or 2, wherein the biodegradable polymer having two or more carboxylic groups at its end is a polymer having an α,α-dicarboxylic group or an α,β,β'-tricarboxylic group at its end.

5. The composition according to claim 1 or 2, wherein the biodegradable polymer having two or more carboxylic groups at its end is a poly α-hydroxycarboxylic acid having an α,α-dicarboxylic group or an α,β,β'-tricarboxylic group at its end.

6. The composition according to claim 5, wherein the poly α-hydroxycarboxylic acid is linear poly α-hydroxycarboxylic acid.

7. The composition according to claim 1 or 2, wherein the biodegradable polymer having two or more carboxylic groups at its end is lactic acid/glycolic acid copolymer having an α,α-dicarboxylic group or an α,β,β'-tricarboxylic group at its end.

8. The composition according to claim 1 or 2, wherein the biodegradable polymer having two or more carboxylic groups at its end is lactic acid/glycolic acid copolymer whose ω residue is tartronic acid or citric acid.

9. The composition according to claim 1 or 2, wherein the biodegradable polymer having two or more carboxylic groups at its end is polylactic acid whose ω residue is tartronic acid or citric acid.

10. The composition according to claim 1 or 2, wherein the physiologically active non-peptide substance is water-insoluble or slightly water-soluble.

11. The composition according to claim 10, wherein the composition is a sustained-release composition.

12. The composition according to claim 11, wherein the physiologically active non-peptide substance has a molecular weight of not more than about 1000.

13. The composition according to claim 11, wherein the physiologically active non-peptide substance is a gonadotropin releasing hormone agonist or antagonist.

14. The composition according to claim 11, wherein the physiologically active non-peptide substance is a compound having a partial structure represented by the formula: 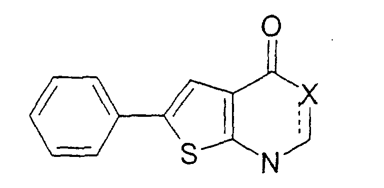 [wherein, X represents a carbon atom or a nitrogen atom, and - - - represents a single bond or a double bond] or a salt thereof.

15. The composition according to claim 14, wherein the composition represented by the formula: 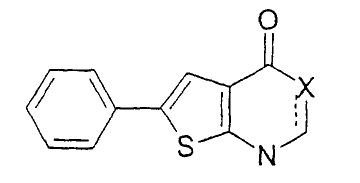 [wherein, X represents a carbon atom or a nitrogen atom, and - - - represents a single bond or a double bond] is a compound represented by the formula: 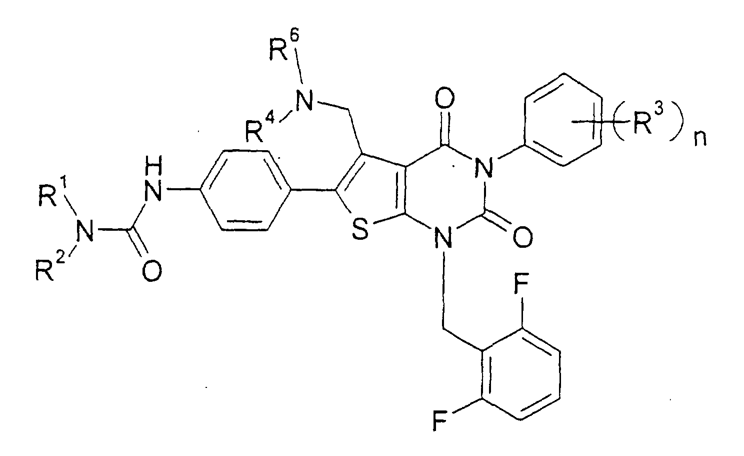 [wherein, R¹ and R² each represent a hydrogen atom, a hydroxy group, a C₁₋₄ alkoxy group, a C₁₋₄ alkoxy-carbonyl group or a C₁₋₄ alkoxy group which may have a substituent,
R³ represents a hydrogen atom, a halogen atom, a hydroxy group or a C₁₋₄ alkoxy group which may have a substituent, or
adjacent two R³s may be linked to form a C₁₋₄ alkylenedioxy group;
R⁴ represents a hydrogen atom or a C₁₋₄ alkyl group;
R⁶ represents a C₁₋₄ alkyl group which may have a substituent or a group represented by the formula: 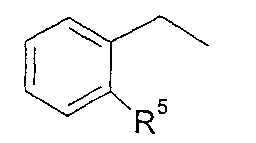 (wherein, R⁵ represents a hydrogen atom or R⁴ and R⁵ may be linked to form a heterocycle); and
n represents an integer of 0 to 5] or a salt thereof.

16. The composition according to claim 14, wherein the composition represented by the formula: 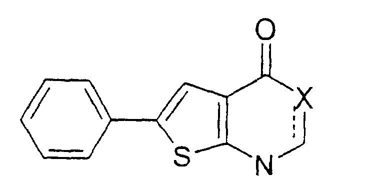 [wherein, X represents a carbon atom or a nitrogen atom, and - - - represents a single bond or a double bond] is 5-(N-benzyl-N-methylaminomethyl)-1-(2,6-difluorobenzyl)-6-[4-(3-methoxyureido)phenyl]-3-phenylthieno[2,3-d]pyrimidine-2,4(1H,3H)-dione or a salt thereof.

17. The composition according to claim 14, wherein the composition represented by the formula: 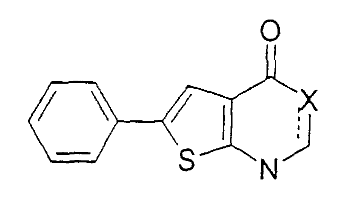 [wherein, X represents a carbon atom or a nitrogen atom, and - - - represents a single bond or a double bond] is a compound represented by the formula: 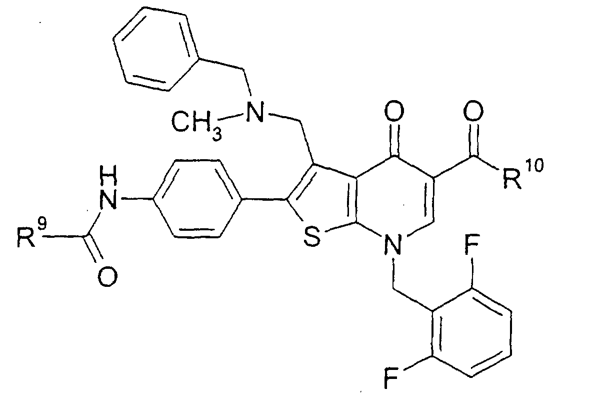 [wherein, R⁹ represents an optionally substituted C₁₋₇ alkyl group, an optionally substituted C₃₋₇ cycloalkyl group, an optionally substituted C₁₋₆ alkoxyamino group, or an optionally substituted hydroxyamino group and
R¹⁰ represents an optionally substituted C₁₋₇ alkyl group or an optionally substituted phenyl group, respectively; or
when R⁹ is an unsubstituted C₁₋₇ alkyl group, R¹⁰ represents a substituted C₁₋₇ alkyl group or a substituted phenyl] or a salt thereof.

18. The composition according to claim 14, wherein the composition represented by the formula: 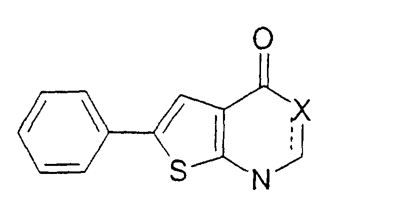 [wherein, X represents a carbon atom or a nitrogen atom, and - - - represents a single bond or a double bond] is 3-(N-benzyl-N-methylaminomethyl)-4,7-dihydro-5-isobutyryl-7-(2,6-difluorobenzyl)-2-[4-[(1-hydroxycyclopropyl)carbonylamino]-phenyl]-4-oxothieno[2,3-b]pyridine or a salt thereof.

19. The composition according to claim 1 or 2, which is used for injection.

20. The composition according to claim 1 or 2, which is in the form of sustained-release microcapsules.

21. A method for producing a composition **characterized by** blending a physiologically active non-peptide substance with a biodegradable polymer having two or more carboxylic groups at its end, or a salt thereof.
